# EUROPEAN PATENT SPECIFICATION

(11) **EP 1 234 187 B1**
(45) Date of publication and mention of the grant of the patent: **30.04.2008**
(21) Application number: 00979297.9
(22) Date of filing: 17.11.2000
(51) Int. Cl.: G06F 19/00

(54) **METHOD, APPARATUS, MEDIA AND SIGNALS FOR IDENTIFYING ASSOCIATED CELL SIGNALING PROTEINS**
VORRICHTUNG, VERFAHREN, MEDIA UND SIGNALE ZUR IDENTIFIZIERUNG VON ASSOZIIERTEN ZELLSIGNALISIERUNGSPROTEINEN
PROCEDE, APPAREIL, SUPPORTS ET SIGNAUX POUR IDENTIFIER LES PROTEINES DE SIGNALISATION CELLULAIRE ASSOCIEES

(30) Priority: 19.11.1999 CA 2290335; 22.11.1999 CA 2290204; 05.07.2000 US 216357 P
(43) Date of publication of application: 28.08.2002
(73) Proprietor: UNIVERSITY OF BRITISH COLUMBIA, Vancouver, British Columbia, V6T 1Z3 (CA)
(72) Inventor: PELECH, Steven, Vancouver, British Columbia V66T 1Z3 (CA)
(74) Representative: Lee, Nicholas John
(86) International application number: PCT/CA2000/001378
(87) International publication number: WO 2001/038879

(56) References cited:
- US-A- 5 914 891
- TAKAI-IGARASHI T AND KAMINUMA T: "A Pathway Finding System for the Cell Signaling Networks Database" IN SILICO BIOLOGY, vol. 1, 1999, pages 129-146, XP002181468

## Description

### FIELD OF THE INVENTION

The present invention relates to analysis of biological information, and more particularly, to methods, apparatus, media and signals for identifying associated cell signaling proteins.

### BACKGROUND OF THE INVENTION

Within a biological cell, levels of expression and activity of proteins are regulated by a subset of typically about **10**% of the proteins, the subset that is dedicated to cell communications and control. This subset is referred to herein as "cell signaling proteins". One of the largest classes of such cell signaling proteins is a class of enzymes called protein kinases. Protein kinases control other proteins by catalyzing their phosphorylation, a process that is reversible by protein phosphatases. Virtually all cell signaling proteins are either protein kinases or regulators of protein kinases or their substrates. Protein kinases often operate within signaling pathways that are further integrated into signaling networks.

Generally, such signaling pathways and networks govern and coordinate all cellular functions, including cell structure, metabolism, reproduction, adaptation, differentiation and death, for example.

Moreover, protein kinases appear to be disproportionately linked to cell diseases. For example, approximately half of the hundred or so identified cancer-inducing genes, or "oncogenes", encode protein kinases, and the remaining half appear to encode proteins that either activate kinases or are phosphorylated by kinases. In addition, over 400 human diseases, such as cardiovascular disease, diabetes, arthritis and other immune disorders, and Alzheimer's disease and other neurological disorders, for example, have been linked to defective signaling through protein kinases.

If protein kinases and their respective signaling pathways and networks can be identified and understood, then monitoring of the kinases could feasibly be used to obtain a molecular diagnosis of a disease condition. In addition, if a particular problematic protein kinase in a diseased cell can be identified, it may be possible to inhibit either the problematic kinase or its downstream effectors, to effectively block the improper proliferative signaling, or even to initiate apototic processes leading to programmed death of the diseased cells such as tumor cells for example.

Therefore, it is particularly important to identify not only the protein kinases and other cell signaling proteins themselves, but more importantly, the signaling pathways and networks in which they operate.

Identification of the kinases themselves is presently being achieved through various genome sequencing projects, and virtually all of the human kinases are expected to be identified within the next year.

However, existing techniques are not suitable for identifying signaling pathways and networks of cell signaling proteins. Most protein kinases appear to be activated as a consequence of either their own phosphorylation by upstream kinases, or by autophosphorylation (i.e. self-phosphoryiation), however, nucleic acid-based techniques such as those used for detection of gene expression cannot be used to monitor post-translational events such as phosphorylation.

Conversely, conventional measurement techniques that are capable of differentiating between phosphorylated and dephosphorylated states of proteins, such as the standard two-dimensional sodium dodecyl sulphate polyacrylamide gel electrophoresis (SDS-PAGE) technique of Dr. Patrick O'Farrell for example, have experienced great difficulty in detecting protein kinases, which are typically present at very minute levels in a cell compared to other proteins. For example, public databases exist containing identifications of over a thousand different proteins on two-dimensional gel maps, but containing identifications of scarcely more than a dozen of the estimated two thousand or so protein kinases that are thought to be encoded by the human genome.

Recent modifications of the two dimensional SDS-PAGE technique involving the Western blotting procedure followed by detection using a single monoclonal anti-kinase antibody per blot, for example, have been attempted. However, the recovery of most protein kinases from the first dimension gel is typically less than **10%,** with the result that **90%** or more of protein kinases do not enter the second dimension gel and are therefore unresolved. Accordingly, these recent modified techniques are typically able to detect only four or five of the several hundred protein kinases that are expected to be present in a given cell.

Due to the above difficulties in obtaining measurements of levels and phosphorylation states of cell signaling proteins such as kinases, few, if any, analytical techniques exist for the analysis of kinase measurements to yield information about the signaling pathways and networks in which the kinases operate.

However, the inventor of the invention disclosed and claimed herein has recently invented a new and useful method for detection of multiple cell signaling proteins, such as multiple kinases or multiple kinase substrates (i.e. proteins that are phosphorylated by kinases), whereby the presence and phosphorylation states of a large number of kinases and/or kinase substrates may be measured in a single sample.

Thus, with the advent of this new experimental measurement technique, there is a need for new analytical techniques for analyzing cell signaling protein measurements, to identify particular cell signaling proteins that are associated with one another in common signaling pathways.

The publication "A Pathway Finding System for the Cell signaling Networks Database" Takako Takai-Igarashi and Tsuguchika Kaminuma, In Silico Biology 1 (1999) 129-146, discloses a knowledge-based pathway-finding system that builds on the cell signaling networks database, CSNDB, and uses a general inference engine to apply rules for finding and coupling pathways between or around specific biomolecules from the CSNDB database. Relationships are found in a large, but fragmented, collection of cell signaling knowledge by filtering out and composing together those sections of pathways specified from an extensive and complex set of binary or pair-wise cell signaling reactions.

A first aspect of the invention provides a method in accordance with claim 1 .

Another aspect of the invention provides apparatus in accordance with claim 32.

Another aspect of the invention provides a computer readable medium for providing instructions for directing a programmable device to execute the method of the first aspect of the invention.

Another aspect of the invention provides a computer data signal embodied in a carrier wave, the signal comprising code segments for directing a programmable device to execute the method of the first aspect of the invention.

Another aspect of the invention provides a computer program comprising code means that when executed by a processor circuit, carries out all the steps of the methods of the first aspect of the invention.

Other aspects and features of the present invention will become apparent to those ordinarily skilled in the art upon review of the following description of specific embodiments of the invention in conjunction with the accompanying figures.

### BRIEF DESCRIPTION OF THE DRAWINGS

In drawings which illustrate embodiments of the invention,
- Figure **1**: is a perspective view of a system for identifying associated cell signaling proteins, according to a first embodiment of the invention;
- Figure **2**: is a block diagram of a processor circuit of an apparatus for identifying associated cell signaling proteins shown in Figure **1****;**
- Figure **3**: is a flowchart of a receive data routine executed by the processor circuit shown in Figure **2****;**
- Figure **4**: is a tabular representation of an input data values store defined in a memory shown in Figure **2****;**
- Figure **5**: is a flowchart of a coexpression routine executed by the processor circuit shown in Figure **2****;**
- Figure **6**: is a tabular representation of a total per protein data store defined in the memory shown in Figure **2****;**
- Figure **7**: is a tabular representation of a first normalized data store defined in the memory shown in Figure **2****;**
- Figure **8**: is a tabular representation of a second normalized data store defined in the memory shown in Figure **2****;**
- Figure **9**: is a tabular representation of a coexpression coefficients store defined in the memory shown in Figure **2****;**
- Figure **10**: is a schematic representation of a coexpressed pairs store defined in the memory shown in Figure **2****;**
- Figure **11**: is a schematic representation of a coexpressed clusters store defined in the memory shown in Figure **2****;**
- Figure **12**: is a flowchart of a clustering subroutine executed by the processor circuit shown in Figure **2****;**
- Figure **13**: is a flowchart of a coregulation routine executed by the processor circuit shown in Figure **2****;**
- Figure **14**: is a tabular representation of a state data store defined in the memory shown in Figure **2****;**
- Figure **15**: is a tabular representation of a coregulation coefficients store defined in the memory shown in Figure **2****;**
- Figure **16**: is a schematic representation of a coregulated pairs store defined in the memory shown in Figure **2****;**
- Figure **17**: is a schematic representation of a coregulated clusters store defined in the memory shown in Figure **2****;**
- Figure **18**: is a flowchart of a linkage routine executed by the processor circuit shown in Figure **2****;**
- Figure **19**: is a tabular representation of a linkage coefficients store defined in the memory shown in Figure **2**;
- Figure **20**: is a tabular representation of a linkage-sorted pairs store defined in the memory shown in Figure **2****;** and
- Figure **21**: is a schematic representation of a linked pathway groups store defined in the memory shown in Figure **2****.**

### DETAILED DESCRIPTION

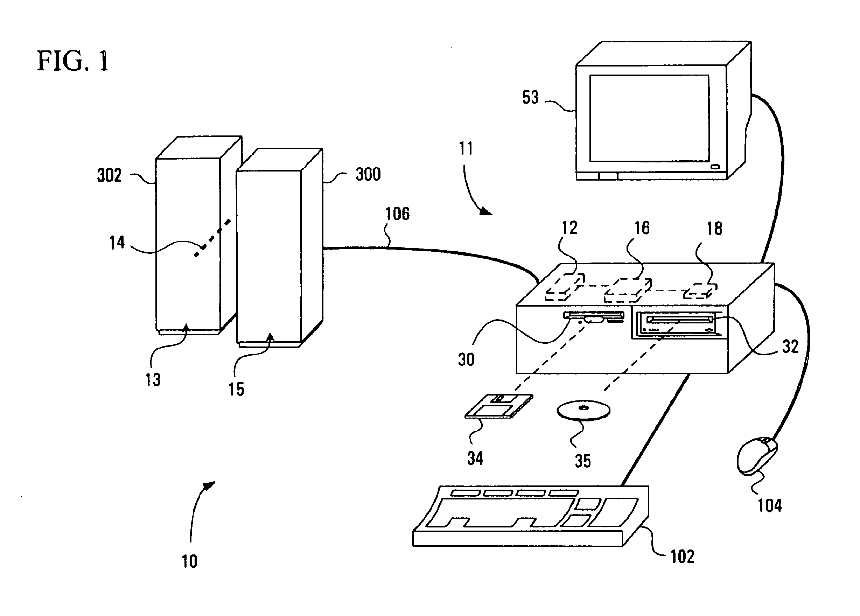

Referring to Figure **1****,** a system and an apparatus for identifying associated cell signaling proteins according to a first embodiment of the invention are shown generally at **10** and **11,** respectively. In this embodiment, the apparatus includes a receiver shown generally at **12,** operable to receive data values representing physical properties of respective cell signaling proteins **14.** The apparatus further includes a processor circuit shown generally at **16** in communication with the receiver **12.** The processor circuit **16** is configured to produce and store, in a memory **18,** a comparison value for each pair of the cell signaling proteins **14** in response to the data values, and to identify cell signaling protein pairs having comparison values satisfying a condition indicative of an association between the cell signaling proteins.

In this specification, including the claims, "cell signaling proteins" means proteins that relay information within cells. Relaying information can include activities such as the binding of an extra-cellular mediator (such as a hormone, growth factor or cytokine for example) to a cell surface receptor, and the cascade of events that mediate the action of that extra-cellular mediator throughout the cell. Examples of cell signaling proteins include receptors, G proteins, second-messenger generating enzymes (such as cyclases, phospholipases and phosphodiesterases), adapter proteins, kinases, phosphatases, apoptosis proteins, heat shock proteins, transcription factors, cyclins, regulatory inhibitors, regulatory activators and scaffolding proteins.

For illustrative purposes, the remainder of this specification describes production and analysis of data representing physical properties of protein kinases. However, one of ordinary skill in the art, upon being presented with this specification, would appreciate that the embodiments of the invention described herein are equally applicable to detection and analysis of other types of cell signaling proteins.

### Processor Circuit

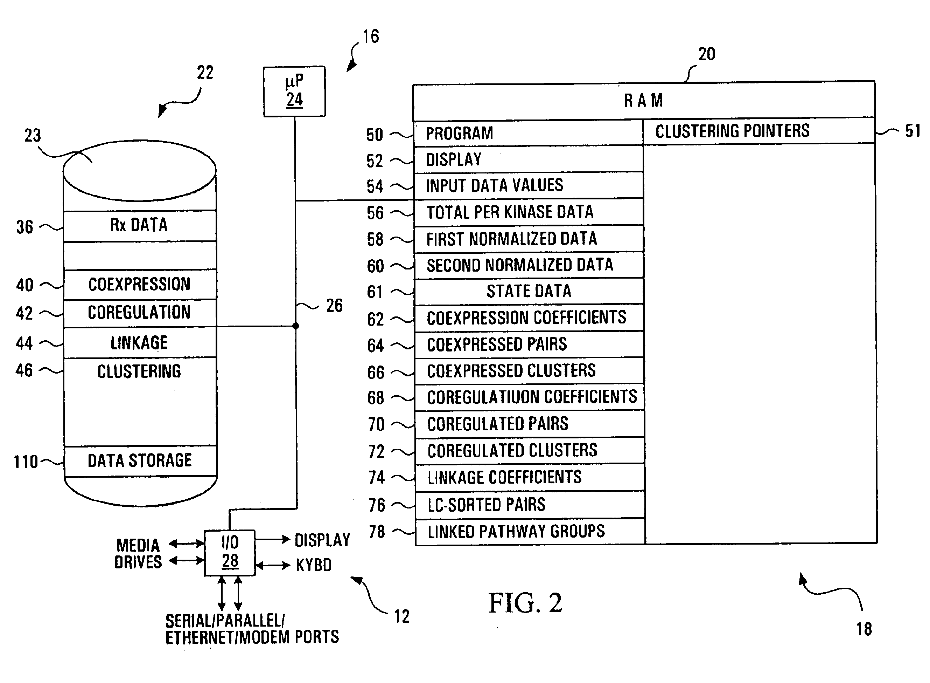

Referring to Figure **2****,** in this embodiment the apparatus **11** includes the memory **18,** which includes a random access memory **20.** The memory **18** further includes a permanent storage medium **22,** which in this embodiment is a hard disk **23.** Alternatively, other types of memory and/or storage media may be substituted.

The processor circuit **16** includes a microprocessor **24,** in communication with the RAM **20,** the hard disk **23** and the receiver **12** via a data bus **26.**

### Receiver

Referring to Figures **1** and **2****,** in this embodiment, the receiver **12** includes an input/output (I/O) unit **28** shown in Figure **2****.** The I/O unit **28** is in communication one or more media drives, such as a floppy diskette drive **30** and a CD-RW drive **32,** for example. The I/O unit is in further communication with a plurality of communication ports, including a serial port, a parallel port, an Ethernet interface and a modem, for example. Thus, receiving values representing physical properties of respective cell signaling proteins **14** may be achieved by receiving, at the I/O unit **28,** data values read by a media drive from a computer-readable medium, such as a floppy diskette **34** for example. Alternatively, such data values may be received at the I/O unit **28** from a local device connected to one of the communications ports, or from a remote device such as an internet-based web server that stores such values, for example.

### Production of Data Values

Data values representing the physical properties of cell signaling proteins may be obtained from preparing samples and employing equipment optionally included in the system shown in Figure **1****.**

Referring back to Figure **1****,** the system **10** may include a measuring device, such as the hardware **15,** operable to produce the data values representing the physical properties of the respective cell signaling proteins. More particularly, in this embodiment the hardware **15** includes a chemiluminescence imager **300** operable to produce signals representative of proteins separated in a single dimension in an electrophoresis gel. In this embodiment the imager **300** includes a Fluor-S Max Multi-imager from Bio-Rad Laboratories Canada Ltd., of Mississauga, Ontario. Canada. The measuring device may further include an electrophoresis apparatus **302** operable to produce the electrophoresis gel. In this embodiment, the electrophoresis apparatus **302** includes a one-dimensional sodium dodecyl sulphate polyacrylamide get electrophoresis (SDS-PAGE) measuring system.

Samples used to be tested for kinase or kinase substrate content may be any cell or tissue homogenate, extract or other such sample which has been processed to purify or partially purify kinases or kinase substrates in the sample. This technique is particularly suitable for testing patient biopsy samples. Such samples may be manipulated to increase prevalence of desired cell types or subcellular fractions in the sample. The sample will be typically prepared for electrophoresis using standard techniques, employing appropriate buffers which may contain various inhibitors or enzymes. For example, protease inhibitors may be present to reduce protein degradation on the sample. Where the sample is to be tested for kinase substrate content, it may be desirable to add one or more protein phosphatases to dephosphorylate substrates which may already exist in a phosphorylated state in the sample. The protein phosphatase will then be inactivated with an appropriate phosphatase inhibitor (e.g. β-glycerophosphate, sodium fluoride or sodium orthovanadate) and a selected protein kinase or mixture of protein kinases is then added to the sample to phosphorylate those substrates present which are specific to kinase added to the sample. Alternatively, endogenous kinases in the sample may be relied upon to phosphorylate dephosphorylated substrates in the sample.

SDS-PAGE employed in this technique is gel electrophoresis performed in a single dimension, typically using a slab shaped gel or a series of tube gels. The gel may be constructed and used employing standard methods, electrophoresis buffers and electrophoresis equipment. The gel may comprise a stacking gel and a separation gel. Commercial kits and equipment are available for performing SDS-PAGE. Preferable contents of the separation gel range from **10%** to **15%** (acrylamide) and **0.2** to **2%** (bisacrylamide). For separation of kinases, an electric current will typically be applied to the gel until proteins with a molecular mass of less than about 25-**27** kDa are eluted from the bottom of the gel as protein kinases do not have a molecular mass less than the latter amount.

Conventional wisdom calls for the use of two dimensional (2D) gel eletrophoresis to monitor changes in protein expression or post-translational modification of proteins. While it is possible to visualize some protein kinases on 2D gels by immunoblotting techniques, the inventor has discovered that in most cases, a maximum of only four or five protein kinases can be detected at a time by Western blotting of 2D gels with mixtures of protein kinase-specific antibodies. Furthermore, recovery of most protein kinases from a first dimension pH gradient gel, is less than 10%. That means that 90% of more of the protein kinases do not enter the second dimension gel and are therefore unresolved. The inventor's attempts to detect multiple kinases by immunoblotting 2D gels of rat brain lysates with multiple antibodies has demonstrated that 2D gel electrophoresis is insufficiently sensitive and inconsistently reproducible for the simultaneous detection of proteins as rare as cell signaling proteins.

Once electrophoresis is complete resulting in a pattern of separated protein moieties in the gel, the pattern is transferred to any membrane (e.g. nitrocellulose, PVDF, nylon, etc.) that is suitable for use in the Western Blotting technique. Transfer is typically done by standard electro-transfer techniques. Once the pattern is transferred to the membrane, the membrane may be cut into strips each of which will typically contain a pattern separated from a single sample (e.g. a test sample or a control sample). Alternatively, the membrane may be left intact for probing with a multiblotting apparatus such as MINIBLOTTER 20 (™), commercially available from lmmunetics Inc. (Cambridge, MA, USA).

Once the electrophoresis and Western blotting aspects of this method are complete, the resulting membrane or membrane strips are probed with a panel of different antibodies that react with distinct categories, subsets, isoforms, etc. of protein kinases or kinase substrates. The panel may be applied in one step as a mixture of antibodies or, the antibodies may be applied sequentially to the membrane. Binding of such antibodies to moieties present on the membrane is then detected using any suitable immunoassay procedure (e.g. see: Stites and Terr (eds) "Basic and Clinical Immunology", (7 ed) 1991**).** A particularly suitable procedure is to treat the antibodies in the panel as primary antibodies in a "sandwich" type assay. Unbound primary antibodies are washed away or otherwise removed. The membrane is then treated with secondary antibodies which are reactive with the primary antibodies. The secondary antibody may be bound to a detectable label or fused with an enzyme. Secondary antibody bound to primary antibody is detected by observing the label or the activity of the fused enzyme. Suitable labels and enzymes are known in the art and include magnetic or colored beads, fluorescent dyes, radiolabels, horseradish peroxidase, alkaline phosphatase, etc. The enzyme linked sandwich type assay (ELISA) is a particularly suitable methodology for use in this technique.

It is preferable that the panel of anti-kinase antibodies comprise polyclonal antibodies rather than MAB's. This departure from conventional wisdom increases the likelihood that new kinase proteins will be detected in the sample since polyclonal anti-kinase antibodies generally exhibit greater cross-reactivity to kinases as compared to anti-kinase MAB's. Despite the use of polyclonal antibodies, the panel may comprise from 2 to about 100 antibodies.

Antibodies for use in the method described herein may be obtained commercially or prepared using standard techniques. A variety of anti-kinase and anti-kinase substrate polyclonal antibodies are commercially available from various sources, including the following:
Biomol Research Laboratories, Inc. (Plymouth Meeting, Pennsylvania)
Biosource International, Inc. (Camarillo, California)
Promega Corporation (Madison, Wisconsin)
Santa Cruz Biotechnology (Santa Cruz, California)
Sigma (Saint Louis, Missouri)
StressGen Biotechnologies Corp. (Victoria, British Columbia)
Transduction Laboratories (Lexington, Kentucky)
Upstate Biotechnology Inc. (Lake Placid, New York)
Zymed Laboratories Inc. (South San Francisco, California)

Antibodies to new kinases may be prepared as described below. Typically, new kinases are partially purified by techniques such as column chromatography and SDS-PAGE. Microsequencing of partially purified kinases permits comparison to known kinases and possible development of immunological techniques for recovery of more of the new kinase by making use of cross reactivity with known antibodies. Antibodies can be raised against protein kinases or substrates in various host animals, including but not limited to cattle, horses, rabbits, goats, sheep and mice. Polyclonal antibodies can be obtained from immunized animals and tested for specificity using standard techniques. Alternatively, monoclonal antibodies may be prepared using any technique that provides for production of antibody molecules by continuous cell lines in culture, including the hybridoma technique of Kohler and Millstein, the human B-cell hybridoma technique, and the EBV-hybridomain technique. Alternatively, techniques for the production of single chain antibodies and antibody fragments that contain specific binding sites for a protein kinase or substrate may be generated by known techniques and employed in this technique. Such fragments include F(ab')**₂** fragments that may be generated by digestion of an intact antibody molecule and Fab fragments that may be generated by severing disulfide bridges in F(ab')**₂** fragments or through the use of Fab expression libraries.

Preferably, none of the antibodies in a given mixture to be used as a panel in this technique will cross-react with proteins that overlap in size. This may compromise interpretation of the experimental findings. Each antibody panel mixture should be blended to avoid such overlaps. Furthermore, every mixture should be adjusted for the concentration of each antibody so that there is optimal detection of the individual target kinases in diverse cell and tissue samples.

Following incubation of the strips with different mixtures of primary antibodies, the strips are incubated with a secondary antibody (e.g. a goat antibody that recognizes rabbit antibody) that reacts with the primary antibody. The secondary antibody is fused with an enzyme (e.g. alkaline phosphatase or horse radish peroxidase) to facilitate detection of the positions of the primary antibody, to which it binds by producing a light emission in an enzymatic reaction. The separate strips may be reassembled to appear in the order of the original membrane. The reassembled membrane may be subjected to enhanced chemiluminescence (ECL) and exposure to x-ray film or detected by a fluorescence imager (e.g. Fluor-S Max Multi-imager from Bio-Rad Laboratories Canada Ltd., of Mississauga, Ontario, Canada). In this indirect manner, the original positions of resolved protein kinases can be visualized as dark bands on a transparent background. The intensity of the bands can be quantized by densitometric analysis. In many cases, quantization of the amounts of a given protein kinases in the upper, phosphorylated form and the lower dephosphorylated form as resolved by SDS-PAGE can provide an accurate measurement of how much of the kinase is in the inactive and active states.

The method described herein offers advantages over standard **2**D gel proteomic methods. This technique can be applied to any cell or tissue sample. No prelabelling with radioisotopes is necessary, because kinase detection is based on immunoreactivity. The technology could be adapted for wide scale diagnostic applications because the patterns of protein kinase expression are stable for periods of up to six hours before an organ is subjected to fractionation and freezing, providing the organ is stored during this time over ice. This procedure can be carried out within two days from start to finish. By contrast, the **2**D gel electrophoresis approach is extremely laborious, much more difficult to render and takes at least twice the time. This method provides the ability to compare multiple samples side by side. Whereas two or more samples can be analyzed on the same **1**D gel, a **2**D gel can only be used for a single sample. It is more difficult to compare two different samples by the **2**D gel route, because of potential variations in the setting up, running and analysis of separate **2**D gels.

One of the reasons why **2**D gel electrophoresis has become the industry standard for proteomic analysis is the remarkable resolving power of the method and potentially thousands of spots can be distinguished on a **2**D gel. Most of these spots, however, are "fuzzy" in appearance and may be overlapping. The method described herein provides much tighter protein bands with a **2-** to **4**-fold better resolution in the SDS-PAGE size-separation dimension. With detection based on immunoreactivity, the background of metabolic enzymes and structural proteins is essentially eliminated. This background is problematic even for **2**D gel maps of phosphoproteins, since a third of all the proteins inside of cells appear to be phosphorylatable.

In one exemplary embodiment, about **50** µg of a control cell extract from untreated or healthy cells is loaded on to a SDS-PAGE gel in odd numbered lanes. In adjacent, even numbered lanes, equivalent amounts of experimental extracts are deposited. The latter samples are from cells that have been treated with a hormone or drug or that have been obtained from diseased tissue. The extracts may be prepared by homogenizing cells in buffer containing a detergent such as **0.5%** Triton X-100^{™} and protein phosphatase inhibitors (to preserve the state of protein phosphorylation in the sample). The extracts are then subjected to ultracentrifugation to remove insoluble matter.

To optimize the detection of protein band shifts, the SDS-PAGE gel is precast with a higher than normal concentration of acrylamide and a lower than normal concentration of bisacrylamide. An electric current is applied to the slab gel until proteins with a molecular mass less than **27,000** Dalton are eluted from the bottom of the gel. The proteins remaining on the slab gel are then electro-transferred on to a nitrocellulose or PVDF membrane that traps the proteins. The membrane is cut into separate strips that each contain samples of the resolved proteins from both control and experimental cell extracts. Each strip is probed with a different mixture of primary antibodies (e.g. from rabbit) that react with a distinct subset peptide or protein substrate by the protein kinase of interest Each reaction is conducted in a separate tube or well of a microtitre plate.

One application of this technique is for the discovery of novel protein kinases. The following strategy should permit the rapid acquisition of protein kinase drug targets. The objective of this approach is to identify those protein kinases that demonstrate increased expression or phosphorylation in association with a disease state or in response to an extracellular signal such as mitogen, drug or stress factor. The approach is based on the following:
1. Antibodies developed for one protein kinase can cross-react with structurally related protein kinases.
2. A band shift of a cross-reactive protein on an immunoblot is due to phosphorylation, and increased phosphorylation is probably associated with activation of the kinase. Greater than **90%** of the known protein kinases are phosphorylated in their active states. One of the exceptions is glycogen synthase kinase-**3**, which is inhibited when it is phosphorylated on serine by protein kinase B. However, activation of glycogen synthase kinase-**3** is still dependent on tyrosine phosphorylation of this kinase.
3. Proteins that cross-react with protein kinase antibodies and also bind to gamma-ATP-agarose beads have a very high probability of being protein kinases. This resin will capture many ATP binding proteins in addition to protein kinases but this procedure can purify kinases by up to **200**-fold.
4. Proteins that autophosphorylate with [γ-**³²**P]ATP following immunoprecipitation with protein kinase antibody are likely to be protein kinases. Most antibodies are unsuitable for immunoprecipitation of proteins, and may require partial denaturation of the proteins. Denatured kinases would have little or no autophosphorylating activity.
**5.** A combination of gamma-ATP-agarose, immunosorbent and fast protein liquid chromatography column steps followed by SDS-PAGE permits rapid purification of an immunoreactive protein to allow for its identification by sequencing.
6. There is a likelihood that a protein kinase detected with antibodies is novel. Of the **2000** or so kinases expected to exist, only about a third have been fully sequenced. Nevertheless, partial cDNA sequences for most protein kinases are available in public and private EST cDNA sequence databases. Once a portion of the cDNA structure of the protein kinase gene is available, it is straightforward to obtain the complete nucleotide and amino acid structures of the gene and its protein with standard methodologies.

One of the beneficial outcomes of this technique is that unknown proteins which can cross-react with the kinase-specific antibodies are detected. Those unidentified proteins that change in their abundance or their phosphorylation state in response to a disease condition or treatment are worthy of closer inspection. If such proteins can be shown to bind to ATP-agarose or to be capable of autophosphorylation with radioactively labeled ATP, then there is a high probability that they are protein kinases. Moreover, it is possible to purify the protein so that it can be sequenced by the Edman degradation method or identified by mass spectroscopy of trypsin digested fragments of the protein. Purification is best done using the antibody that was originally used to detect the putative kinase. If any part of the protein has been previously sequenced, it would be available in public or private protein sequence databases. A partial sequence in the human EST sequence database may be available. From this information, a full length cDNA sequence for the protein could be rapidly obtained using PCR-based techniques. This would be worthwhile if the cDNA sequence contained conserved kinase catalytic subdomain sequences. In this manner, novel protein kinases that display desirable characteristics (e.g. increased expression in solid tumor relative to adjacent, normal tissue) can be detected and identified, If the inappropriate activity of such protein kinase is shown to contribute to the development of the disease, then they would be most valuable drug targets.

Initial detection of measurement of the activation of a protein kinase in the methods described herein is dependent on the detection of its band shift on SDS-PAGE gels. Phosphorylated forms often appear to be **0.5** to **2** kDa larger than their dephosphorylated counterparts. In a small number of cases, phosphorylation is indicative of inactivation of a kinase. A limited number of protein kinases do not exhibit a band shift change when they are activated. However, their *in vivo* substrates can display band shifts upon their phosphorylation. This can be exploited for the development of *in vitro* and *in vivo* substrate assays. Current approaches for high throughput screening of protein kinase inhibitors *in vitro* involve the use of radioactive [γ-**³²**]ATP and measurement of the incorporation of the radioactive phosphate into a peptide or protein substrate by protein kinase of interest. Each reaction is conducted in a separate tube or well of a microtitre plate generating high volumes of radioactive garbage.

There are many examples of proteins that are highly specific substrates of particular protein kinases; examples include glycogen phosphorylase for phosphorylase kinase, myosin light chain for myosin light chain kinase, elF**2**α for PKR, MARCKS for protein kinase C, Erk**1** and Erk**2** for Mek**1** and Mek**2**. Antibodies are commercially available for many of these substrates or may be produced as described above. Such antibodies may be used to probe for the phospho-states of the substrates, as revealed by their mobility on immunoblots of SDS-PAGE gels. These substrates would not have to be purified from crude cellular extracts for use in the protein kinase assays. However, since many of the substrates may already exist in phosphorylated forms in cell extracts, it may be necessary to incubate the extracts with active preparations of protein phosphatases, which can be subsequently inactivated with phosphatase inhibitors prior to the kinase assays. With this method, a crude mixture of active protein kinases may be added to the phosphatase-treated cellular extract in a single tube, and the phosphorylation reaction can commence with the inclusion of non-radioactive ATP. Only catalytic amounts of protein kinases will be necessary, so any phosphorylated substrates that contaminate the preparation of protein kinases will be relatively minor compared to the amounts of the substrates in the phosphatase-treated cell extracts. Any kinases that contaminate the phosphatase-treated cell extracts would not be a concern, since they are actually desirable. It may be necessary to add a kinase preparation after phosphatase treatment of the substrate extracts, because many protein kinases are inhibited when they are dephosphorylated. After a short suitable incubation time, the reactions can be terminated by addition of SDS-PAGE sample buffer. Such substrate analysis can be performed as described above for protein kinases, except that panels of antibodies for the kinase substrates will be used in place of the kinase antibody panels. By this approach, the decreased mobility of the kinase substrates will be evident as band shift on the immunoblots in the absence of kinase inhibitors. The presence of specific protein kinase inhibitors would be revealed by the inhibition of the appearance of the upper bands.

*In vitro* substrate analysis according to the methods described herein would be ideal for the further characterization of compounds that have already been shown to display inhibitor activity toward a kinase and the selectivity of these compounds is in question. A distinct advantage of this method is that it would be easy to compare the findings with a substrate analysis *in vivo* assay performed using the same blends and concentrations of kinase substrate antibodies that work in the *in vitro* kinase assay. However, the analysis would be performed on extracts from cells that have been incubated with agonists that stimulate the kinases of interest. These cells would also be exposed to the compounds that exhibit inhibitory activity towards kinase *in vitro.* In this manner, the efficacy of these inhibitors could be evaluated in living cells.

The intensity of the signal that is generated for a protein kinase band may be readily quantified using known technologies. For example, quantification may be done by using a Fluor-S Max Multi-imager from Bio-Rad Laboratories Canada Ltd., of Mississauga, Ontario, Canada. This equipment can quantize changes in band intensity in range of **1:100,000** but **1:1,000** is more typical. Multiple exposures of X-ray films to ECL for detection of immunoreactive bands to compensate for any non-linearity of response of the film prior to quantization by densitometric analysis could be performed as an alternative method.

Each immunoreactive band is assigned a set of parameters that includes its relative optical density, molecular weight and immunoreactivity. The relative optical density (R.O.D.) value of an immunoreactive protein band is based on the ratio of the intensity of that protein relative to the intensity of a protein kinase band that serves as an internal control. For example, the mitogen-activated protein (MAP) kinase Erk**1** in **50** µg of rat brain cytosolic protein detected with Erk1-CT antibody could serve as such an internal control. Erk**1** has been found to be one of the most uniformly expressed protein kinases in different rat tissues and diverse organisms. Alternative standards could be the zeta isoform of protein kinase C or the alpha isoform of p**38** Hog MAP kinase. If a protein has the same intensity on a Western blot as Erk**1** in rat brain, then it has an R.O.D. value of **100**.

Another parameter is the molecular mass of an immunoreactive protein band, which is based on its migration on the SDS-PAGE gel relative to known molecular mass marker proteins such as phosphorylase, bovine serum albumin, ovalbumin, glyceraldehyde **3**-phosphate dehydrogenase and lysozyme.

A further parameter is the immunoreactivity of a protein band, which is somewhat selective, and particularly appropriate when the immunogen to which the antibody was originally developed is considered. For example, an antibody developed against the C-terminal **40** amino acids of the rat brain Erk**1** isoform would be expected to immunoreact with the full-length **44** kDa form of Erk**1** on Western blots of rat brain cytosol.

Determination of the structure of a protein kinase network is based on the following principles.
i. Modules of protein kinases have been highly conserved in the evolution of diverse eukaryotes. These modules mediate the transmission of information in signaling pathways.
ii. Protein kinases that operate within a common module would be typically coexpressed in cells found in different tissues and species.
iii. Protein kinase networks are formed from the interconnection of diverse protein kinase modules. This implies extensive crosstalk between signaling modules.
iv. An upward shift in a protein kinase band due to reduced mobility reflects its regulation, most likely marking its activation. If other protein kinase bands are similarly affected by the same set of cell stimuli, then they may operate within the same module.
v. The higher the correlation between two protein bands with respect to their coexpression and their band shifting in response to a wide diversity of stimuli, the closer that they operate within a common signaling module. Protein kinases that are not coexpressed and do not undergo coregulation, are not connected in the same pathway.

Determination of the architecture of a protein kinase network requires the examination of a wide range of different cell types and perturbations. Ideally, at least one hundred different experimental model systems should be analyzed. It is important that while extremely diverse model systems are explored, the methods and probes for the Western blotting analysis are uniformly consistent. By testing the regulation of the same group (e.g. **100**) of different protein kinases in a several diverse model systems with a wide range of different perturbations, each immunoreactive band can be separately compared with each of the other hundred or more immunoreactive bands for

### (1) the intensity of the signals and (2) whether the protein kinases are similarly altered in their phosphorylated states.

It is noteworthy that values representing physical properties of kinases have not been readily available in the past. Accordingly, the above description illustrates a method of detecting multiple kinases or multiple kinase substrates, to obtain data values representing physical properties of kinases, in the event that such data cannot be conveniently received from commercial sources such as internet-based web servers, compact discs or other media, for example. Once the procedures described above have been performed, to produce a one-dimensional electrophoresis gel such as that shown at **13** in Figure **1****,** existing hardware **15** and software (not shown) may be used to measure the gel to produce digital data values representing the physical properties of the kinases, such as their amounts in phosphorylated and dephosphorylated states respectively, for example. Such software preferably produces such digital data and stores it on the floppy diskette 34 or other medium, in a MICROSOFT EXCEL (™) spreadsheet table format readable by EXCEL **98** (™) software from Microsoft Corporation, Redmond, Washington, USA, however, other formats may be substituted. An example of suitable software for densitometric quantization of chemiluminescence generated by ECL from a Western blot of target proteins is the QUANTITY ONE (™) software from Bio-Rad Laboratories Canada Ltd., of Mississauga, Ontario, Canada.

### Configuration of data processing system

Referring to Figure **2****,** in this embodiment the storage medium **22** acts as a computer readable medium for providing instructions for directing a programmable device to perform various functions described herein. In this regard, the storage medium **22** stores a plurality of routines, each routine including blocks of instruction codes which configure or program the processor circuit **16** to perform such functions. Alternatively, such blocks of instruction codes may be obtained as segments of a signal embodied in a carrier wave received at the processor circuit. One group of the blocks of instruction codes, for example, provides a receive data routine **36** which configures the processor circuit **16** to cooperate with the receiver **12** to receive data values representing physical properties of respective cell signaling proteins **14.**

A coexpression routine **40** configures the processor circuit to produce, as the comparison values, a coexpression coefficient for each pair of the cell signaling proteins, each coexpression coefficient representing a degree of coexpression of one cell signaling protein of the pair and the other cell signaling protein of the pair. The coexpression routine also configures the processor circuit to normalize the data values relative to at least one reference value, prior to producing the comparison values.

A coregulation routine **42** configures the processor circuit to produce, as the comparison values, a coregulation coefficient for each pair of the cell signaling proteins, each coregulation coefficient representing a degree of coregulation of one cell signaling protein of the pair and the other cell signaling protein of the pair.

A linkage routine **44** configures the processor circuit to produce, as the comparison values, a linkage coefficient for each pair of the cell signaling proteins, as a function of a coexpression coefficient representing a degree of coexpression of one cell signaling protein of the pair and the other cell signaling protein of the pair, and of a coregulation coefficient representing a degree of coregulation of the one cell signaling protein of the pair and the other cell signaling protein of the pair, each linkage coefficient representing a degree of association between the one cell signaling protein and the other cell signaling protein.

A clustering subroutine **46** configures the processor circuit to identify a cluster of associated cell signaling proteins, by identifying a group of pairs of associated cell signaling proteins for which each member of each pair is present in at least one other pair of the group.

The various instruction codes stored in the storage medium **22** further configure the processor circuit **16** to define, in the RAM **20,** a plurality of buffers, registers and stores. For example, referring to Figures **1** and **2****,** the processor circuit **16** is directed to define in the RAM **20,** a program store **50** for temporarily storing the various routines or portions thereof for execution by the processor circuit and for providing a temporary calculation area, a clustering pointers register **51** for storing pointers for use by the clustering subroutine **46,** and a display buffer **52** for storing data representing an image to be displayed on a display such as that shown at **53** in Figure **1****.**

Referring again to Figure **2****,** the processor circuit **16** is further directed to define the following additional stores in the RAM **20.** An input data values store **54** is used to store received data values representing physical properties of respective cell signaling proteins, which in this embodiment are kinases. A total per protein (TPP) data store **56** is used to store data representing total amounts of respective cell signaling proteins (in this embodiment, kinases), irrespective of the phosphorylation states of such signaling proteins. First and second normalized data stores **58** and **60** are used to store data representing normalized cell signaling protein data values. A state data store **61** stores data representing phosphorylation states of the cell signaling proteins. A coexpression coefficients store **62** is used to store a coexpression coefficient for each pair of the cell signaling proteins. A coexpressed pairs store **64** stores a list of coexpressed cell signaling protein pairs, and a coexpressed clusters store **66** stores a list of clusters of the coexpressed cell signaling protein pairs. Similarly, a coregulation coefficients store **68** is used to store a coregulation coefficient for each pair of the cell signaling proteins, a coregulated pairs store **70** stores a list of coregulated cell signaling protein pairs, and a coregulated clusters store **72** stores a list of clusters of the coregulated cell signaling protein pairs. A linkage coefficients store **74** stores a linkage coefficient for each pair of the cell signaling proteins. A linkage-sorted pairs store **76** stores a list of cell signaling protein pairs sorted by their linkage coefficients, and a linked pathway groups store **78** stores a plurality of groups of cell signaling proteins associated with common signaling pathways.

### Operation

Generally, the present embodiment of the invention involves producing and storing a comparison value for each pair of cell signaling proteins in response to data values representing physical properties of respective cell signaling proteins, such as the amounts or quantities of the cell signaling proteins detected in respective biological samples, and/or phosphorylation states of the cell signaling proteins, for example. Cell signaling protein pairs having comparison values satisfying a condition indicative of an association between the cell signaling proteins are then identified.

A number of different techniques may be used to produce the comparison values, with the result that a number of corresponding conditions indicative of associations between the cell signaling proteins may be applied.

For example, if a first kinase tends to be present whenever a second kinase is present, such kinases are likely to be associated. Thus, one way of producing comparison values includes producing a coexpression coefficient for each pair of the cell signaling proteins, representing a degree of coexpression of one cell signaling protein of the pair and the other cell signaling protein of the pair.

Similarly, if two kinases tend to be found in the same phosphorylation state, such kinases are likely to be commonly regulated and are therefore also likely to be associated with each other. Thus, a second way of producing comparison values includes producing a coregulation coefficient for each pair of the cell signaling proteins, representing a degree of coregulation of one cell signaling protein of the pair and the other cell signaling protein of the pair.

Either of the above methods of producing comparison values may be used by itself, independently of the other. However, the present embodiment of the invention further provides a third method of producing comparison values, by combining the results of the coexpression and coregulation analyses, to produce a linkage coefficient for each cell signaling protein pair as a function of the coexpression and coregulation coefficients for the pair. Using the linkage coefficients, cell signaling proteins may then be associated with respective common signaling pathways.

Alternatively, other methods of producing comparison values may be apparent to one of ordinary skill in the art upon reading this specification and are not considered to depart from the scope of the present invention.

### Receive data routine

Referring to Figures **2****,** **3** and **4****,** the operation of the present embodiment of the invention begins with execution of the receive data routine **36** by the processor circuit **16.** The receive data routine **36** includes a plurality of blocks of instruction codes that configure or program the microprocessor **24** to cooperate with the I/O unit **28** to act as a receiver operable to receive data values representing physical properties of respective cell signaling proteins. More particularly, in this embodiment the microprocessor and I/O unit cooperate to receive sets of cell signaling protein data, each set including data values representing amounts of respective corresponding cell signaling proteins in biological material corresponding to the set, and further including data values indicative of phosphorylation states of respective cell signaling proteins in biological material corresponding to the set. Although it is preferable to receive such data, indicative of both the amounts and the phosphorylation states of the cell signaling proteins, alternatively, significant benefits may be achieved even if the data is indicative of only one of these two exemplary physical properties.

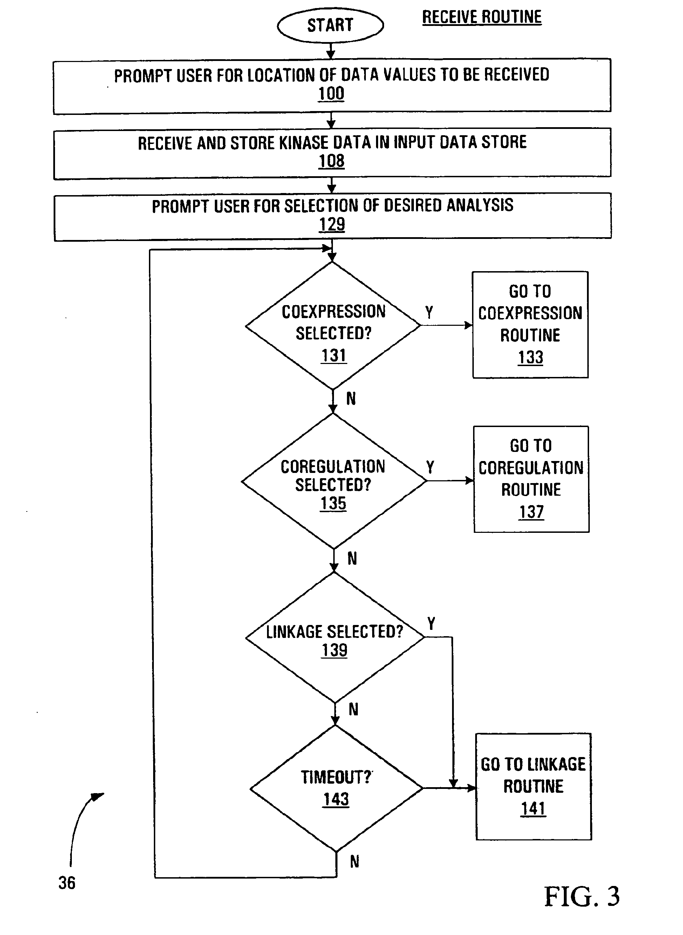

Referring to Figures **1****,** **2** and **3****,** the receive data routine **36** begins with a first block of codes **100** shown in Figure **3****,** which directs the processor circuit **16** to control the display **53** to prompt a user of the apparatus **11** to enter information representing a location from which the desired kinase data values or other cell signaling protein data values may be received. Such information may be entered by the user via a user interface device such as a keyboard **102** or a mouse **104** shown in Figure **1****,** for example. The information may include a file path, such as a:/kinasedata.xis or d:/kinasedata.xis for example, indicating that the data values are to be received from an EXCEL (™) spreadsheet file stored on either the floppy diskette **34** or a compact disc **35,** via the floppy diskette drive **30** or the CD-RW drive **32** respectively. Or, if the user has chosen to actually produce the desired data values, the information may include an identification of the hardware **15** to direct the processor circuit to receive the data values directly from the hardware **15,** via a cable **106** such as a parallel port cable for example, connecting the hardware to the I/O unit **28.** Alternatively, the information may include any other location information, such as a Universal Resource Locator (URL) or an Internet Protocol (I.P.) address for example, indicating a location on a network such as the Internet from which the data values may be downloaded via the I/O unit **28.**

After receiving user input providing such location information, block 108 directs the microprocessor **24** to cooperate with the I/O unit **28** to receive the data values from the specified location, such as an EXCEL (™) spreadsheet file stored on the floppy diskette **34** for example, and to store the data values in the input data values store **54** in the RAM **20.** In this embodiment, block **108** further directs the processor circuit **16** to copy the newly-stored contents of the input data values store **54** to a data storage area **110** in the storage medium **22** for permanent storage therein, in the event that subsequent re-analysis of the data values may be desired at a later date.

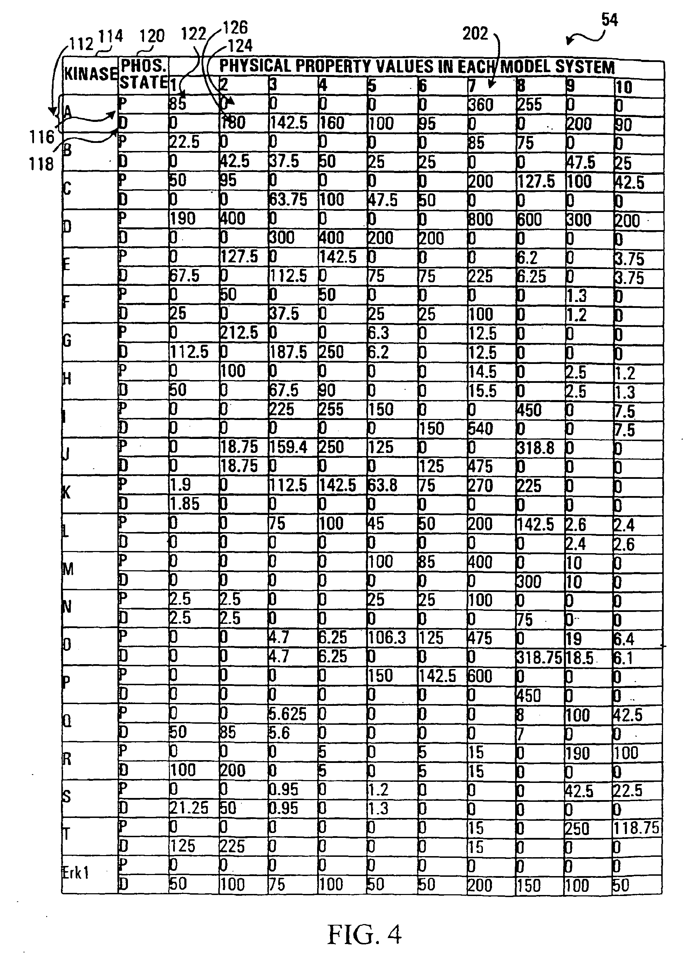

Referring to Figure **4****,** the structure and contents of the input data values store immediately following execution of block **108** are illustrated generally at **54.** For each cell signaling protein, a cell signaling protein data record **112** is defined, having an identification field **114** containing an identification of the particular cell signaling protein, such as "A", "B" or "Erk**1**", for example. The remainder of the cell signaling protein data record **112** is divided into a phosphorylated sub-record **116** and a dephosphorylated sub-record **118.** Each of the sub-records **116** and **118** includes a plurality of information fields. A phosphorylation state field **120** contains an identification of a phosphorylation state of the cell signaling protein, such as "P" or an active bit to indicate a phosphorylated state, or a "D" or an inactive bit to indicate a dephosphorylated state, for example. Each sub-record then contains a plurality of measurement fields **122,** each measurement field corresponding to a particular set of data values or measurements of all cell signaling proteins identified in the input data values store **54.**

For illustrative purposes, in this embodiment only ten sets of data values corresponding to ten respective measurements of the cell signaling proteins in ten respective biological samples are received and analyzed, however, it will be appreciated that a much larger number of measurements, such as **100** separately measured model systems for example, is desirable in order to improve the statistical reliability of the results. Therefore, in this embodiment each phosphorylated sub-record **116** includes ten phosphorylated measurement fields **124,** each containing a measurement of an amount of the cell signaling protein detected in a phosphorylated state, and similarly, each dephosphorylated sub-record **118** includes ten dephosphorylated measurement fields **126,** each containing a measurement of an amount of the cell signaling protein detected in a dephosphorylated state.

Alternatively, if a user desires to obtain only coexpression information, without coregulation or linkage information, each cell signaling protein data record **112** may contain only a single measurement field **122** for each measurement set or model system, the measurement field **122** containing a measurement of a total detected amount of the corresponding cell signaling protein, irrespective of its phosphorylation state. Conversely, if a user desires to obtain only coregulation information, without coexpression or linkage information, each cell signaling protein data record **112** may contain only a single measurement field **122** for each measurement set or model system, the measurement field **122** containing an indication of the phosphorylation state in which the corresponding cell signaling protein was detected, irrespective of the amount that was detected.

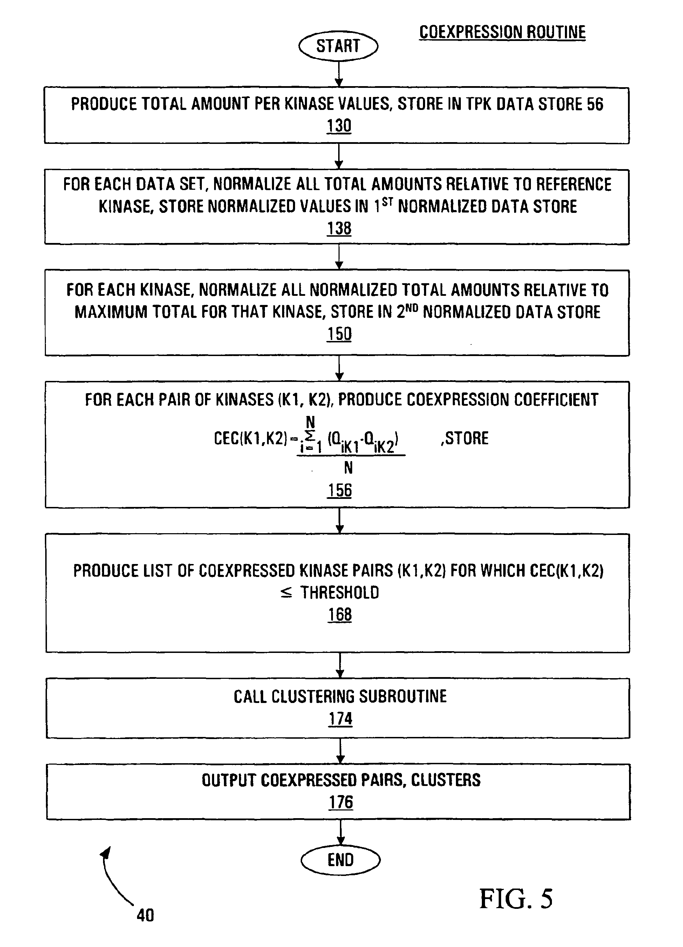

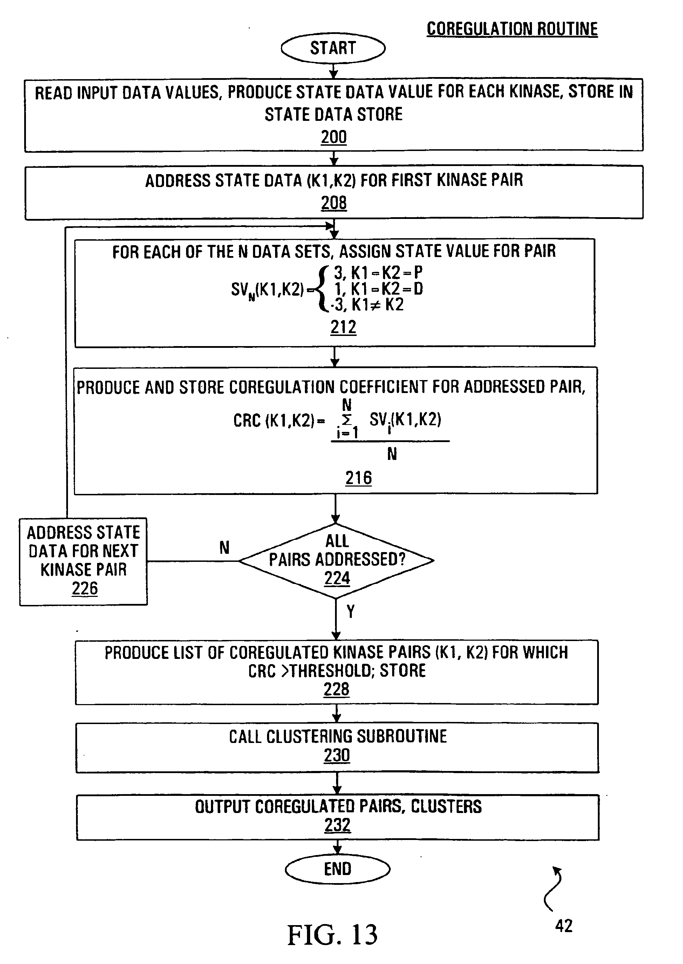

Referring back to Figure **3****,** following such receipt and storage of data values at block **108,** block **129** directs the processor circuit **16** to control the display **53** to prompt the user of the apparatus **11** to select a desired analysis, such as coexpression, coregulation or linkage analysis, for example. Block **131** then directs the processor circuit **16** to determine whether user input selecting coexpression has been entered, in which case block **133** directs the processor circuit to execute the coexpression routine **40** shown in Figure **5****.** Referring back to Figure **3****,** similarly, block **135** directs the processor circuit to determine whether user input selecting coregulation has been entered, in which case block **137** directs the processor circuit to execute the coregulation routine **42** shown in Figure **13****.** Referring back to Figure **3****,** if at block **139** the processor circuit determines that user input selecting linkage has been entered, block **141** directs the processor circuit to execute the linkage routine **44** shown in Figure **18.** Referring back to Figure **3****,** similarly, if at block **143** the processor circuit has failed to receive any user input within a pre-defined timeout period, the processor circuit is directed to execute the linkage routine **44** by default. If the timeout period has not elapsed, processing continues through blocks **129** to **143.** Following such direction at either block **133, 137** or **141,** the receive data routine **36** is ended.

### Coexpression Routine

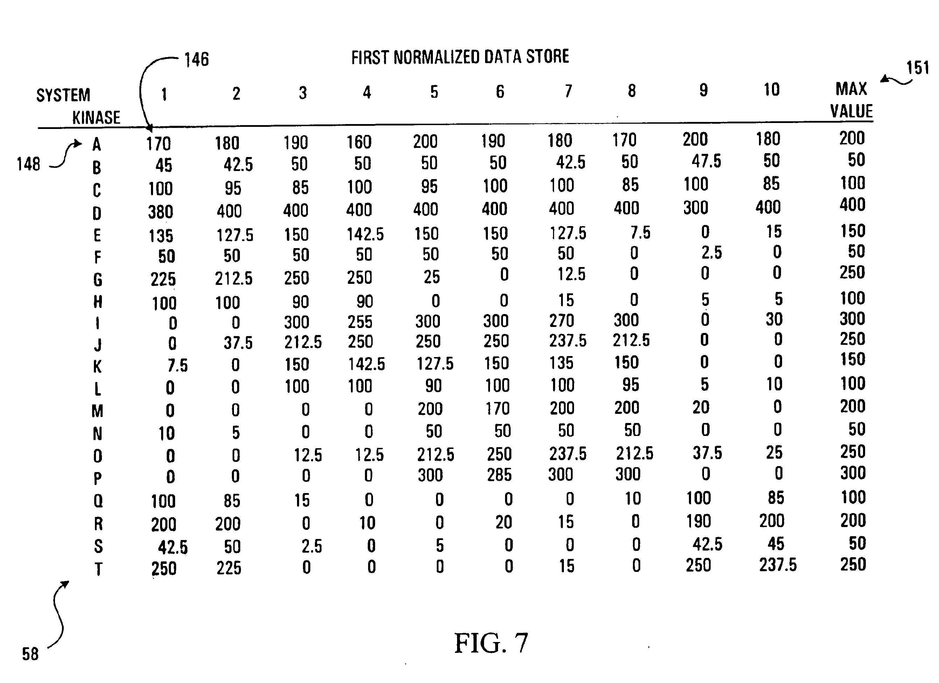

Referring to Figures **5** to **11****,** the coexpression routine is shown generally at **40** in Figure **5****.** Generally, the coexpression routine **40** configures the processor circuit **16** to produce and store, in the memory **18,** a comparison value for each pair of the cell signaling proteins in response to the received cell signaling protein data values, and to identify cell signaling protein pairs having comparison values satisfying a condition indicative of an association between the cell signaling proteins. More particularly, the coexpression routine configures the processor circuit to produce, as the comparison values, a coexpression coefficient for each pair of the cell signaling proteins, each coexpression coefficient representing a degree of coexpression of one cell signaling protein of the pair and the other cell signaling protein of the pair.

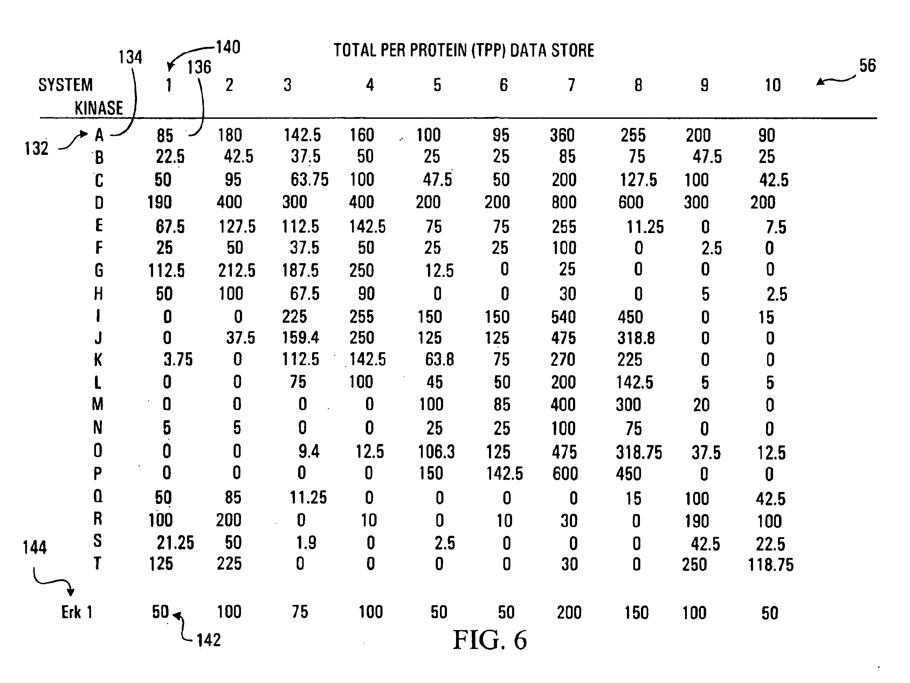

Referring to Figures **2****,** **4****,** **5** and **6****,** the coexpression routine **40** begins with a first block of codes shown at **130** in Figure **5****,** which directs the processor circuit **16** to read the contents of the input data values store **54** shown in Figure **4****,** and to use such contents to produce total amount per protein values and store such values in the total per protein (TPP) data store **56** shown in Figure **6****.**

Referring to Figure **6****,** in this embodiment the TPP data store **56** contains a plurality of protein total amount records **132.** Each protein total amount record **132** includes an identification field **134** for storing an identification of the cell signaling protein, and a plurality of total amount fields **136,** each total amount field **136** containing a number representing a total detected amount of the cell signaling protein in a particular corresponding measurement set or model system.

Referring to Figures **4****,** **5** and **6****,** block **130** directs the processor circuit **16** to sequentially address each cell signaling protein data record **112** stored in the input data values store **54.** For each such addressed cell signaling protein data record **112,** block **130** directs the processor circuit to sequentially address each set of data measurements. For each such addressed set, block **130** directs the processor circuit to add the contents of the phosphorylated measurement field **124** and the dephosphorylated measurement field **126** of the currently addressed cell signaling protein data record **112,** and to store this sum in a total amount field **136** shown in Figure **6****,** corresponding to the currently addressed set of data measurements, of a protein total amount record **132** corresponding to the currently addressed cell signaling protein data record **112.**

Referring to Figures **5****,** **6** and **7****.** block **138** then configures the processor circuit to normalize the values stored in the TPP data store **56,** relative to at least one reference value, prior to producing the comparison values. It will be appreciated that the experimental sensitivity of each measurement set or model system may vary from set to set. Accordingly, it is desirable to normalize each set of cell signaling protein data by expressing each data value in the set relative to a particular reference value in the set that corresponds to a cell signaling protein or other detected protein that is expected to be present in a roughly identical amount in all measurement sets or model systems.

Thus, referring to Figure **6****,** for each data set **140** in the TPP data store **56,** the contents of each total amount field **136** of the data set **140** are expressed relative to the contents of a reference measurement field **142** of a reference total amount record **144.** More particularly, in this embodiment, the reference total amount record **144** includes ten reference measurement fields **142,** one for each data set **140,** the contents of each reference measurement field **142** representing a total amount of Erk**1**, detected with an Erk**1**-CT antibody. To achieve this relative expression, block **138** directs the processor circuit **16** to divide each amount stored in each total amount field **136** of a particular data set **140,** by one percent of the contents of the reference measurement field **142** of that data set **140.** For example, if the contents of the reference measurement field **142** for the data set are equal to 200, the contents of each total amount field **136** of that data set are divided by two.

Referring to Figures **2****,** **5** and **7****,** block **138** further directs the processor circuit **16** to store such normalized values in corresponding once-normalized measurement fields **146** of corresponding once-normalized cell signaling protein data records **148** in the first normalized data store **58.**

Referring to Figures **2****,** **5****,** and **7** and **8,** block **150** then directs the processor circuit **16** to further normalize the total amount values by expressing each total amount for a given cell signaling protein as a. percentage of the maximum amount of that particular cell signaling protein detected in any of the ten measurement sets. To achieve this, block **150** first directs the processor circuit to successively address each once-normalized cell signaling protein data record **148** in the first normalized data store **58.** For each such addressed record **148,** block **150** directs the processor circuit to produce a maximum normalized amount value equal to the maximum quantity stored in any of the ten once-normalized measurement fields **146** of the particular once-normalized cell signaling protein data record **148.**

Block **150** further directs the processor circuit to store this maximum value in a maximum normalized amount field **151** of the once-normalized cell signaling protein data record **148.** Block **150** then directs the processor circuit to divide the contents of each once-normalized measurement field **146** of the currently addressed record **148** by one percent of the contents of the maximum normalized amount field **151** of the addressed record **148.** Block **150** further directs the processor circuit to store the resulting quantity in a twice-normalized measurement field **152** of a twice-normalized cell signaling protein data record **154** in the second normalized data store **60,** the record **154** corresponding to the currently addressed once-normalized cell signaling protein data record **148.** Block **150** continues to successively address once-normalized cell signaling protein data records **148** in this manner until such twice-normalized values have been produced and stored in each twice-normalized measurement field **152** of each twice-normalized cell signaling protein data record **154.**

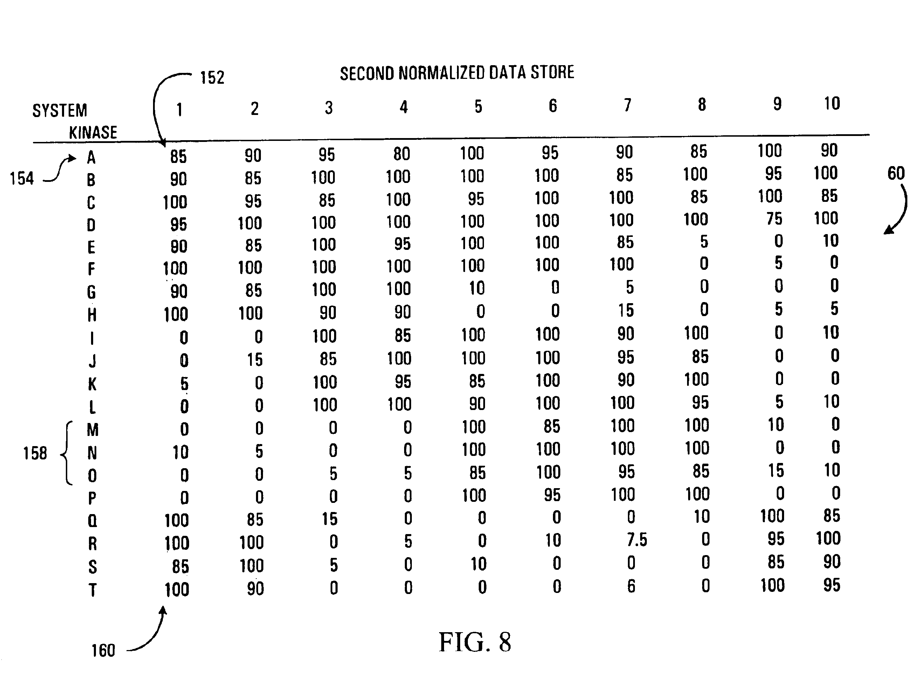

Referring to Figures **2****,** **5****,** **8** and **9****,** block **156** then directs the processor circuit **16** to produce, as the comparison values, a coexpression coefficient for each pair of the cell signaling proteins, each coexpression coefficient representing a degree of coexpression of one cell signaling protein of the pair and the other cell signaling protein of the pair. To achieve this, block **156** configures the processor circuit to successively address each possible pair of cell signaling proteins identified in respective twice-normalized cell signaling protein data records **154** stored in the second normalized data store **60,** such as an exemplary cell signaling protein pair **158** shown in Figure **8** consisting of the kinases identified as "M" and "O", for example. For each such addressed cell signaling protein pair **158,** block **156** configures the processor circuit to calculate, for each of the ten data sets **160** in the second normalized data store **60,** a difference value equal to an absolute value of a difference between a data value stored in the twice-normalized measurement field **152** corresponding to one cell signaling protein of the addressed pair **158,** and a data value stored in the twice-normalized measurement field **152** corresponding to the other cell signaling protein of the addressed pair **158.**

Once all ten such difference values for the ten respective data sets **160** have been calculated for the currently addressed pair **158,** block **156** further configures the processor circuit to add the ten difference values for each of the sets to produce a sum of difference values. Block **156** then configures the processor circuit to divide this sum by the number of the sets (in this embodiment, ten) to produce the coexpression coefficient corresponding to the one cell signaling protein and the other cell signaling protein of the currently addressed cell signaling protein pair **158.**

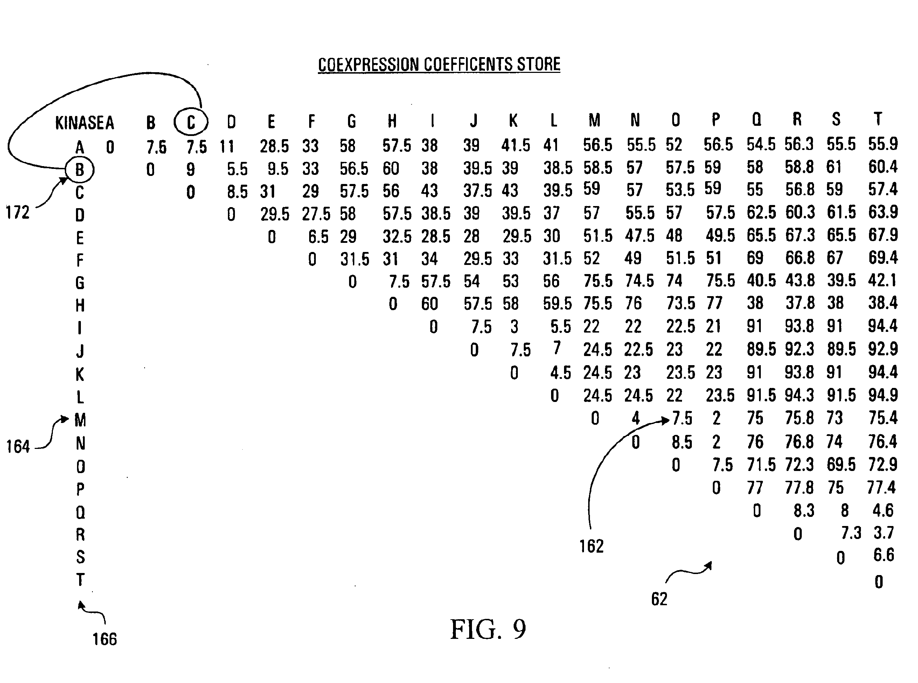

Referring to Figures **5** and **9****,** block **156** further directs the processor circuit **16** to store this coexpression coefficient in the coexpression coefficients store **62,** or more particularly, in a coexpression coefficient field **162** of a cell signaling protein coexpression record **164** corresponding to the first cell signaling protein of the currently addressed pair **158.** As shown in Figure **9****,** each cell signaling protein coexpression record **164** includes a cell signaling protein identification field **166,** and further includes a plurality of coexpression coefficient fields **162,** containing a plurality of respective coexpression coefficients corresponding to the coexpression of the cell signaling protein identified in the identification field **166** and every other successive cell signaling protein (e.g. every kinase whose corresponding coexpression record **164** appears beneath the current coexpression record **164** in the coexpression coefficients store **62** shown in Figure **9****).** It will be appreciated that for the purpose of analyzing coexpression, it is unnecessary to treat the cell signaling protein pairs as permutations rather than combinations, as the coexpression of kinase M with O for example is necessarily identical to the coexpression of O with M. However, if desired, each coexpression record **164** may redundantly contain coexpression coefficients for the identified cell signaling protein and all other cell signaling proteins, rather than merely successive proteins. Block **156** continues to direct the processor circuit to produce and store coexpression coefficients in this manner, until a coexpression coefficient has been produced and stored for every possible pair of cell signaling proteins. Finally, block **156** directs the processor circuit to produce and store, in the data storage area **110** of the storage medium **22,** a copy of the coexpression coefficients store **62,** in the event that it is desired to subsequently retrieve such information.

Referring to Figures **2****,** **5****,** **9** and **10****,** block **168** configures the processor circuit **16** to identify cell signaling protein pairs having comparison values satisfying a condition indicative of an association between the cell signaling proteins, and to produce a list of pairs of associated cell signaling proteins. More particularly, block **168** configures the processor circuit to identify the cell signaling protein pairs by identifying each cell signaling protein pair having a coexpression coefficient less than or equal to a threshold coexpression value. In this embodiment, where the cell signaling proteins are kinases, for the purpose of coexpression, the condition indicative of an association between two kinases is satisfied if the coexpression coefficient for those two kinases is less than or equal to **15.**

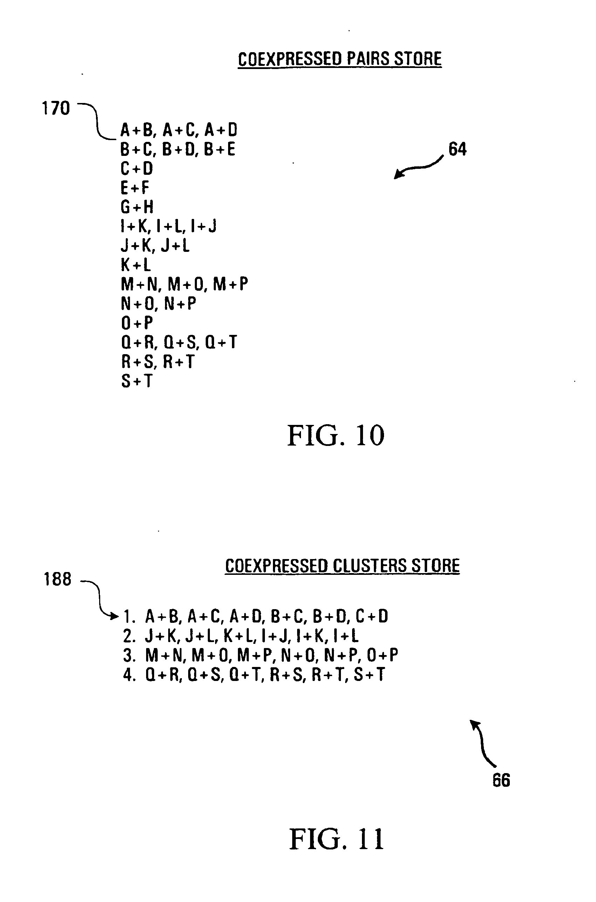

Thus, block **168** configures the processor circuit **16** to read the contents of each of the coexpression coefficient fields **162** in the coexpression coefficients store **62,** and to produce a list of coexpressed cell signaling protein pairs such as that shown in Figure **10****,** the list including an identification of each cell signaling protein pair having a coexpression coefficient less than or equal to the threshold coexpression value, which in this embodiment is **15.** Alternatively, other threshold values may be substituted. As an illustrative example, as shown in Figure **10****.** block **168** directs the processor circuit to include in the list of coexpressed pairs, an identification **172** of an exemplary cell signaling protein pair **170** shown in Figure **9** consisting of the kinases B and C, for which the coexpression coefficient is **9.** Block **168** further directs the processor circuit to store the list of identifications of coexpressed pairs in the coexpressed pairs store **64** shown in Figure **2****,** and to store a copy of the coexpressed pairs store **64** in the data storage area **110** of the storage medium **22** for subsequent retrieval, if desired.

Block **174** then directs the processor circuit **16** to call the clustering subroutine **46,** and to store, in the clustering pointers register **51,** respective pointers to the coexpressed pairs store **64** and the coexpressed clusters store 66, to indicate that the input data to the clustering subroutine is stored in the coexpressed pairs store, and that the output of the clustering subroutine is to be written to the coexpressed clusters store.

Finally, block **176** directs the processor circuit **16** to output the list of coexpressed cell signaling protein pairs stored in the coexpressed pairs store **64,** as shown in Figure **10****,** and the list of coexpressed cell signaling protein clusters stored in the coexpressed clusters store **66,** a shown in Figure **11****.** Such output may include generating a printout of these lists on a printer (not shown) in communication with the processor circuit via the I/O unit **28** for example, and/or displaying such lists on the display **53** shown in Figure **1****.** Alternatively, such output may include writing the contents of the coexpressed pairs store and the coexpressed clusters store to a medium such as the floppy diskette **34** shown in Figure **1**, via the I/O unit **28** and the floppy diskette drive **30,** or to other media, or may include communicating such contents to a remote device via a network such as an intranet or the Internet. Alternatively, other types of outputs may be substituted.

It will be appreciated that the output contents of the coexpressed pairs store **64** and the coexpressed clusters store **66,** as shown in Figures **10** and **11****,** suggest the following conclusions. Kinases A, B, C and D, which form a common cluster, are commonly coexpressed and may operate together in a signaling pathway. Kinases I*,* J, K and L, which also form a common cluster, are commonly coexpressed and may operate together in a signaling pathway. Kinases M, N, O and P, which also form a common cluster, are commonly coexpressed and may operate together in a signaling pathway. Similarly, Kinases Q, R, S and T, which also form a common cluster, are commonly coexpressed and may operate together in a signaling pathway. Kinases E and F are commonly coexpressed and may operate together in a signaling pathway. Kinases G and H are commonly coexpressed and may operate together in a signaling pathway.

The coexpression routine **40** is then ended.

### Clustering Subroutine

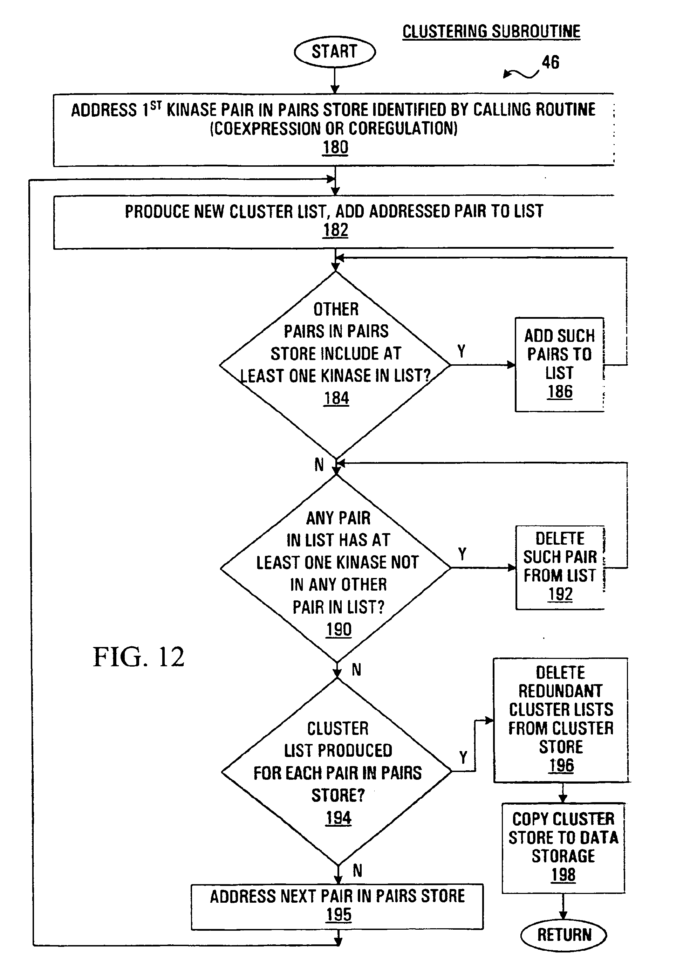

Referring to Figures **2****,** **11** and **12****,** the clustering subroutine **46** configures the processor circuit to produce a list of clusters of associated cell signaling proteins. More particularly, the clustering subroutine configures the processor circuit to identify a cluster of associated cell signaling proteins, the cluster comprising a group of the pairs of associated cell signaling proteins for which each member of each pair is present in at least one other pair of the group.

The clustering subroutine **46** may be called by either the coexpression routine **40,** or the coregulation routine **42,** or both. The calling routine stores appropriate pointers in the clustering pointers register **51** in the RAM **20,** to provide the clustering subroutine **46** with a pointer to an input area of the RAM **20** where input data for the clustering subroutine is located, and a pointer to an output area of the RAM **20** to which the clustering subroutine is to write its output. For example, when the calling routine is the coexpression routine **40,** the clustering pointers register **51** will contain an input pointer to the coexpressed pairs store **64** and an output pointer to the coexpressed clusters store **66.** In this example, therefore, the clustering subroutine configures the processor circuit to produce a list of clusters of coexpressed cell signaling protein pairs, each cluster including a group of the pairs of coexpressed cell signaling proteins for which each member of each pair is present in at least one other pair of the group.

The clustering subroutine begins with a first block of codes **180,** which directs the processor circuit **16** to address a first cell signaling protein pair in a pairs store (in this example, the coexpressed pairs store **64)** identified by the contents of the clustering pointers register **51.**

Block **182** then configures the processor circuit to generate a new cluster list associated with the currently addressed cell signaling protein pair, the cluster list including an identification of the addressed cell signaling protein pair. Block **182** further directs the processor circuit to store the new cluster list in an output area identified by a pointer in the clustering pointers register **51,** which in this example is the coexpressed clusters store **66.**

Blocks **184** and **186** then effectively configure the processor circuit **16** to add, to the new cluster list, an identification of each of the cell signaling protein pairs in the input area, which in this example is the coexpressed pairs store **64,** that includes at least one cell signaling protein already present in the cluster list. Block **184** directs the processor circuit to determine whether any such pairs exist. If so, block **186** directs the processor circuit to add identifications of such pairs to the new cluster list. Block **186** then directs the processor circuit back to block **184** to determine whether the input area contains any cell signaling protein pairs that include at least one cell signaling protein already present in the revised cluster list. In effect, therefore, blocks **184** and **186** configure the processor circuit to repeat the adding following each adding of pairs to the cluster list, to effectively add to the cluster list each of the pairs that includes at least one cell signaling protein present in at least one pair added to the cluster list. Processing continues through blocks **184** and **186** in this manner until no such further pairs exist.

For example, referring to Figures **10, 11** and **12****,** if the currently addressed cell signaling protein pair consists of the kinases A+B, then following block **182,** a new cluster list will include only an identification of the pair A+B. Following an initial execution of blocks **184** and **186,** the new cluster list **188** will further include identifications of any pairs in the coexpressed pairs store **64** that include either A or B, or in other words, the cluster list **188** will consist of the pairs A+B, A+C, A+D, B+C, B+D and B+E. Following a second execution of blocks **184** and **186,** the new cluster list will further include identifications of any pairs that include either C, D or E, or in other words, the new cluster list **188** will consist of A+B, A+C, A+D, B+C, B+D, B+E, C+D and E+F. Upon a third execution of block **184,** no further pairs will be located and the processor will be directed to block **190.**

Blocks **190** and **192** then configure the processor circuit to eliminate from the new cluster list, each of the pairs that includes at least one cell signaling protein not found in at least one other pair in the cluster list. Block **190** directs the processor circuit to determine whether any such pairs exist, and if so, block **192** directs the processor circuit to eliminate such pairs from the new cluster list and to return to block **190** to determine whether any such pairs exist in respect of the revised list. In effect, therefore, blocks **190** and **192** configure the processor circuit to repeat the eliminating following each eliminating of pairs, to effectively eliminate from the cluster list each of the pairs that includes at least one cell signaling protein not present in at least one other non-eliminated pair in the cluster list.

For example, a first execution of blocks **190** and **192** upon the cluster list **188** consisting of A+B, A+C, A+D, B+C, B+D, B+E, C+D and E+F, will result in the elimination of E+F from the new cluster list, as F is not found in at least one other pair in the cluster list **188.** A second execution of blocks **190** and **192** upon the revised list will result in the further elimination of B+E, as E is no longer found in at least one other non-eliminated pair in the cluster list **188.** Upon a further execution of block **190,** no such pairs will be found, leaving a revised cluster list **188** consisting of A+B, A+C, A+D, B+C, B+D and C+D.

Following the final execution of block **190,** block **194** then directs the processor circuit to determine whether a cluster list such as the cluster list **188** has been produced for each cell signaling protein pair in the input area, which in this example is the coexpressed pairs store **64.** If not, block **195** directs the processor circuit to address the next cell signaling protein pair in the coexpressed pairs store **64** and the processor is directed back to block **182,** to generate a new cluster list corresponding to the newly-addressed cell signaling protein pair.

If at block **194,** the processor circuit determines that a cluster list has been produced for each cell signaling protein pair, block **196** then directs the processor circuit to compare the various cluster lists **188** stored in the output area, which in this example is the coexpressed clusters store **66,** to each other, to determine whether any of the cluster lists **188** are identical to each other. For example, it will be appreciated that in the exemplary cluster lists illustrated in Figure **11****,** a cluster list corresponding to C+D will consist of the same cluster list **188** as that corresponding to A+B. Block **196** therefore directs the processor circuit to delete any such redundant copies of cluster lists from the output area (in this example the coexpressed clusters store **66**), so that each cluster appears only once as a combination of pairs, rather than multiple times as a permutation of pairs.

Block **198** then directs the processor circuit to store, in the data storage area **110** of the storage medium **22,** a copy of the output area, i.e. a copy of the coexpressed clusters store **66.**

Following execution of block **198,** the processor circuit **16** is directed to return to whichever routine called the clustering subroutine **46.**

### Coregulation Routine

Referring to Figures **2** and **13** **- 17,** the coregulation routine is shown generally at **42** in Figure **13****.** Generally, the coregulation routine **42** configures the processor circuit **16** to produce and store, in the memory **18,** a comparison value for each pair of the cell signaling proteins in response to the received cell signaling protein data values, and to identify cell signaling protein pairs having comparison values satisfying a condition indicative of an association between the cell signaling proteins. More particularly, the coregulation routine configures the processor circuit to produce, as the comparison values, a coregulation coefficient for each pair of the cell signaling proteins, each coregulation coefficient representing a degree of coregulation of one cell signaling protein of the pair and the other cell signaling protein of the pair.

Referring to Figures **2****,** **4****,** **13** and **14****,** the coregulation routine **42** begins with a first block of codes **200,** which directs the processor circuit **16** to generate state data corresponding to the contents of the input data values store **54,** and to store such state data in the state data store **61.** Block **200** directs the processor circuit to sequentially address each cell signaling protein data record **112** in the input data values store **54,** and for each addressed cell signaling protein data record, the processor circuit is directed to sequentially address each of the ten data sets **202** of the input data values store **54.**

Block **200** further directs the processor circuit to read the contents of the phosphorylated measurement field **124** and the dephosphorylated measurement field **126** corresponding to the currently addressed data set **202** and the currently addressed cell signaling protein data record **112.** If the contents of the phosphorylated measurement field **124** are greater than or equal to both the contents of the dephosphorylated measurement field **126** and a predetermined value, which in this embodiment is **20,** then block **200** directs the processor circuit to store an indication of a phosphorylated state, such as an active bit or a "P" for example, in a state data field **204** of a cell signaling protein state record **206** in the state data store **61,** corresponding to the currently addressed cell signaling protein data record **112** and data set **202.** Conversely, if the contents of the dephosphorylated measurement field **126** are greater than the contents of the phosphorylated measurement field **124** and are greater than or equal to the predetermined value, then an indication of a dephosphorylated state, such as an inactive bit or a "D" for example, is stored in the corresponding state data field **204** of the cell signaling protein state record **206.** If the contents of the phosphorylated measurement field **124** and the dephosphorylated measurement field **126** are both less than the predetermined value, then block **200** directs the processor circuit to store in the state data field **204** an indication that no reliable conclusion as to the phosphorylation state of the cell signaling protein may be drawn for this particular data set, such as a string "NS" representing "No Signal", for example.

Alternatively, rather than merely distinguishing between phosphorylated and dephosphorylated states in a binary manner, for some applications it may be desirable to distinguish between partial degrees of phosphorylation. For example, it may be desirable to assign an indication "D" representing a dephosphorylated state when the amount of the cell signaling protein detected in the dephosphorylated state, expressed as a percentage X_{D} of the total detected amount of the cell signaling protein, falls in the range 90%≤X_{D}≤100%, to assign an indication P**1** of a first phosphorylated state when 60%≤X_{D}<90%, to assign an indication P**2** of a second phosphorylated state when 30%≤X_{D}<60%, and to assign an indication P**3** of a third phosphorylated state when 0%≤X_{D}<30%.

In this embodiment, block **200** directs the processor to continue producing and storing two-state (P or D) phosphorylation state data, until a cell signaling protein state record **206** has been produced and stored for all cell signaling proteins, each state record including a state data field **204** for each of the ten data sets.

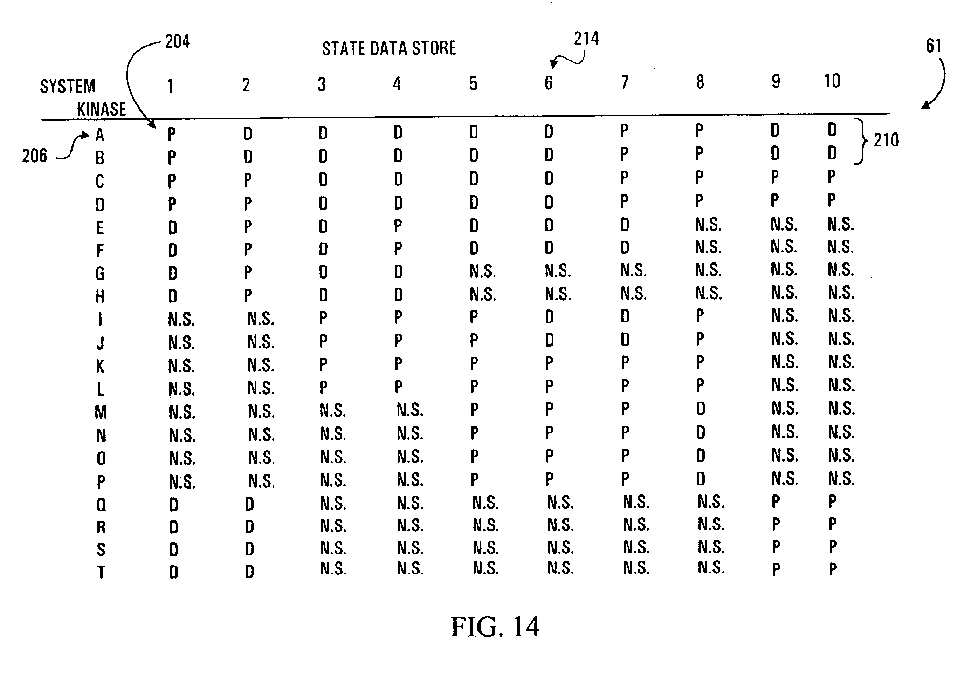

Referring to Figures **2****,** **13** and **14****,** block **208** then directs the processor circuit **16** to address the cell signaling protein state records **206** corresponding to a first cell signaling protein pair, such as an exemplary cell signaling protein pair **210** shown in Figure **14****,** consisting of the kinases A and B.

Block **212** then configures the processor circuit to assign, for each of the ten data sets, a pair state value, as a function of phosphorylation states of one cell signaling protein and the other cell signaling protein of the currently addressed cell signaling protein pair. Any suitable function may be used, but preferably, the selection of the function for producing the pair state values reflects the following three principles. If both members of a particular pair of kinases or other cell signaling proteins are consistently found in a phosphorylated state, this is strongly suggestive of a coregulation association between the kinases. If both members of the pair are consistently found in a dephosphorylated state, this still suggests a coregulation association, but may alternatively be explained by the coincidental fact that in most situations, the majority of kinases are in dephosphorylated states, and therefore, the likelihood of coregulation is not as high as if they were both found to be phosphorylated. Conversely, if both members of the pair are frequently found in opposite phosphorylation states, this strongly suggests that the members are not coregulated.

Thus, in this embodiment, to reflect these three principles, block **212** configures the processor circuit **16** to assign such pair state values by assigning a first pair state value when the one cell signaling protein and the other cell signaling protein of the pair are both in a phosphorylated state, by assigning a second pair state value when the one cell signaling protein and the other cell signaling protein are both in a dephosphorylated state, the second pair state value being less than the first pair state value, and by assigning a third pair state value when the one cell signaling protein and the other cell signaling protein are in different phosphorylation states, the third pair state value being less than the second pair state value. More particularly, in this embodiment the first pair state value is **+3,** the second pair state value is **+1,** and the third pair state value is **-3.** If, for any of the ten data sets **214,** the state data field **204** of the cell signaling protein state record **206** of either of the cell signaling proteins of the currently addressed pair contains a value indicative of an unreliable phosphorylation state determination, such as "N.S." for example, block **212** directs the processor circuit to omit producing any pair state value for that particular data set **214** for the currently addressed pair. Block **212** directs the processor circuit to temporarily store the pair state values produced for the currently addressed cell signaling protein pair, in a pair state calculation area (not shown) in the program store **50** in the RAM **20.**

Block **216** then configures the processor circuit **16** to produce and store a coregulation coefficient corresponding to the one cell signaling protein and the other cell signaling protein of the currently addressed cell signaling protein pair. Block **216** first directs the processor circuit to add the pair state values for each of the sets, as produced at block **212,** to produce a sum of pair state values corresponding to the currently addressed cell signaling protein pair. Block **216** further configures the processor circuit to divide the sum by the number of sets, to produce the coregulation coefficient corresponding to the one cell signaling protein and the other cell signaling protein. This division is a division by the number of sets for which pair state values were actually produced for the pair, which may differ from the total number of data sets if any of the state data fields **204** contained "N.S." indicating unreliable phosphorylation state data. If the coregulation coefficient is negative, in this embodiment block **216** directs the processor circuit to set the coregulation coefficient equal to zero. Alternatively, however, such coregulation coefficients may be left as negative values if desired.

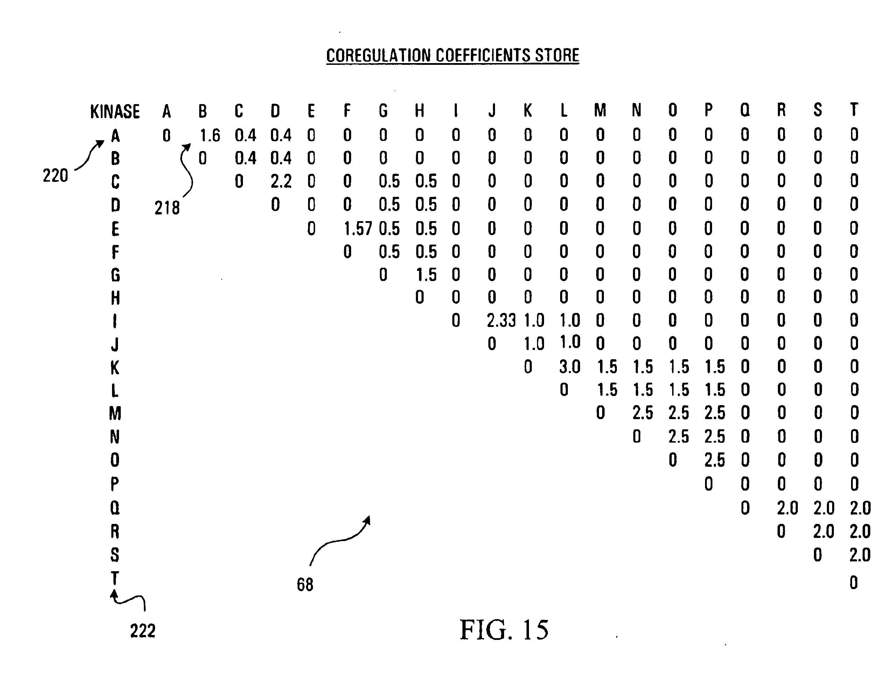

Referring to Figures **13** and **15****,** block **216** further directs the processor circuit to store the coregulation coefficient in the coregulation coefficients store **68,** or more particularly, in a coregulation coefficient field **218** of a cell signaling protein coregulation record **220** corresponding to the first cell signaling protein of the currently addressed pair **210.** As shown in Figure **15****,** each cell signaling protein coregulation record **220** includes a cell signaling protein identification field **222,** and further includes a plurality of coregulation coefficient fields **218,** containing a plurality of respective coregulation coefficients corresponding to the coregulation of the cell signaling protein identified in the identification field **222** and every other successive cell signaling protein (e.g. every kinase whose corresponding coregulation record **220** appears beneath the current coregulation record **220** in the coregulation coefficients store **68** shown in Figure **15****).** It will be appreciated that for the purpose of analyzing coregulation, it is unnecessary to treat the cell signaling protein pairs as permutations rather than combinations, as the coregulation of cell signaling protein B with cell signaling protein A for example is necessarily identical to the coregulation of A with B. However, if desired, each coregulation record **220** may redundantly contain coregulation coefficients for the identified cell signaling protein and all other cell signaling proteins, rather than merely successive proteins.

Block **224** then directs the processor circuit **16** to determine whether pair state values and coregulation coefficients have been produced for all possible cell signaling protein pairs, and if not, block **226** directs the processor circuit to address the cell signaling protein state records **206** in the state data store **61** corresponding to the next cell signaling protein pair, and to return to block **212** to continue assigning pair state values and producing coregulation coefficients, as described above.

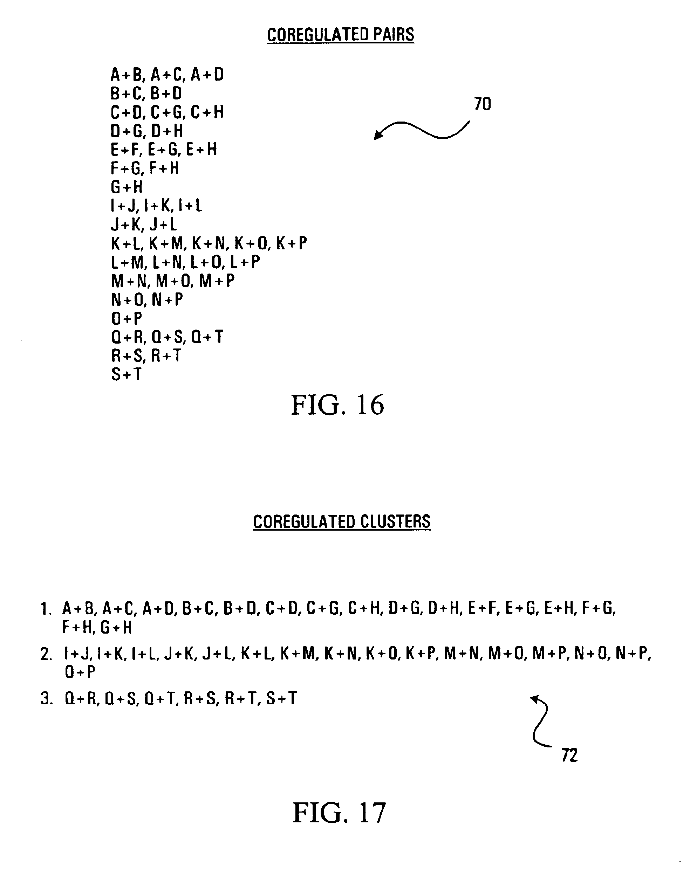

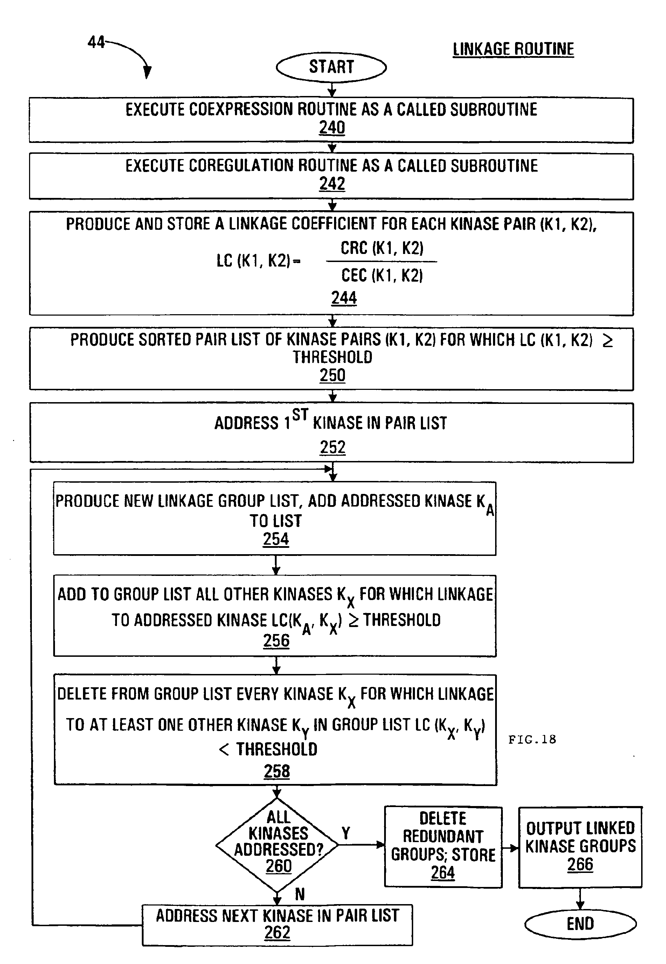

Referring to Figures **13****,** **15** and **16****,** if at block **224,** coregulation coefficients have been produced for all cell signaling protein pairs, block **228** configures the processor circuit **16** to identify cell signaling protein pairs having comparison values satisfying a condition indicative of an association between the cell signaling proteins, and to produce a list of such pairs of associated cell signaling proteins. More particularly, block **228** configures the processor circuit to identify and produce a list of coregulated cell signaling protein pairs, the list including an identification of each cell signaling protein pair having a coregulation coefficient greater than a threshold coregulation value. In this embodiment, the threshold coregulation value is zero, so that any pairs having coregulation coefficients that are negative or equal to zero will be omitted from the list. Alternatively, other threshold values may be substituted. Block **228** further directs the processor circuit to store the list of coregulated pairs so identified in the coregulated pairs store **70** in the RAM **20,** as shown at **70** in Figure **16****.** In addition, block **228** directs the processor circuit to store copies of the coregulation coefficients store **68** and the coregulated pairs store 70 in the storage area **110** of the storage medium **22,** to enable subsequent retrieval if desired.

Referring to Figures **2** and **13****,** block **230** then directs the processor circuit **16** to call the clustering subroutine **46,** to produce a list of clusters of coregulated cell signaling protein pairs, each cluster including a group of the coregulated cell signaling protein pairs for which each member of each pair is present in at least one other pair of the group. Block **230** further directs the processor circuit to store, in the clustering pointers register **51,** an input area pointer identifying the coregulated pairs store **70** as the location of input data for the clustering subroutine, and an output area pointer identifying the coregulated clusters store **72** as the location to which the clustering subroutine is to write its output.

Referring to Figures **2****,** **12****,** **16** and **17****,** the clustering subroutine **46** then directs the processor circuit **16** to analyze the contents of the coregulated pairs store shown at **70** in Figure **16****,** and to produce and store, as the contents of the coregulated clusters store **72** shown in Figure **17****,** a list of clusters of coregulated cell signaling protein pairs, each cluster including a group of coregulated cell signaling protein pairs for which each member of each pair is present in at least one other pair of the group. The production of such a coregulated clusters list proceeds in precisely the same manner as described above in connection with the production of the contents of the coexpressed clusters store **66,** and is therefore not described in further detail.

Block **232** then directs the processor circuit **16** to output the contents of the coregulated pairs store **70** and the coregulated clusters store **72.** As with the output of coexpressed pairs and clusters, such output may include generating a printout or a display, or writing to a computer-readable medium, or transmitting the output to a remote device, for example.

It will be appreciated that the output contents of the coregulated pairs store **70** and the coregulated clusters store **72** shown in Figures **16** and **17** respectively, suggest the following conclusions. Kinases A, B, C, D, E, F, G and H are commonly coregulated and may operate in common signaling pathways. Kinases I, J, K, L, M, N, O and P are commonly coregulated and may operate in common signaling pathways. Similarly, kinases Q, R, S and T are commonly coregulated and may operate in common signaling pathways.

The coregulation routine **42** is then ended.

### Linkage Routine

Referring to Figure **18,** the linkage routine is shown generally at **44.** Generally, the linkage routine **44** configures the processor circuit **16** to produce and store, in the memory **18,** a comparison value for each pair of cell signaling proteins in response to the received data values, and to identify cell signaling protein pairs having comparison values satisfying a condition indicative of an association between the cell signaling proteins. More particularly, in this embodiment the linkage routine configures the processor circuit to produce, as the comparison values, a linkage coefficient for each pair of the cell signaling proteins, as a function of a coexpression coefficient representing a degree of coexpression of one cell signaling protein of the pair and the other cell signaling protein of the pair, and of a coregulation coefficient representing a degree of coregulation of the one cell signaling protein of the pair and the other cell signaling protein of the pair, each linkage coefficient representing a degree of association between the one cell signaling protein and the other cell signaling protein.

Referring back to Figure **3****,** it will be recalled that the linkage routine is invoked either in response to an express user request detected at block **139** of the receive data routine **36,** or alternatively, is invoked as a default in the absence of any routine selection by the user.

Accordingly, referring to Figures **2** and **18,** the linkage routine **44** begins with a first block of codes **240** which directs the processor circuit **16** to execute the coexpression routine **40** as a called subroutine, in order to produce and store the contents of the coexpressed pairs store **64** and the coexpressed clusters store **66** as discussed above. Similarly, block **242** directs the processor circuit to execute the coregulation routine **42** as a called subroutine, in order to produce and store the contents of the coregulated pairs store **70** and the coregulated clusters store **72** as discussed above.

Referring to Figures **2****,** **9****,** **15****, 18** and **19****,** block **244** then configures the processor circuit **16** to produce each linkage coefficient by, for each pair, dividing the coregulation coefficient by the coexpression coefficient. More particularly, for each pair of cell signaling proteins, block **244** directs the processor to read the contents of the coregulation coefficient field **218** in the coregulation coefficients store **68** corresponding to the pair, and to divide such contents by the contents of the coexpression coefficient field **162** in the coexpression coefficient store **62** corresponding to the pair. For the purpose of such division, if the contents of the coexpression coefficient field **162** are zero, block **244** directs the processor circuit to instead divide the coregulation coefficient by an arbitrary small number, such as **1x10⁻⁴** for example, to avoid division-by-zero errors.

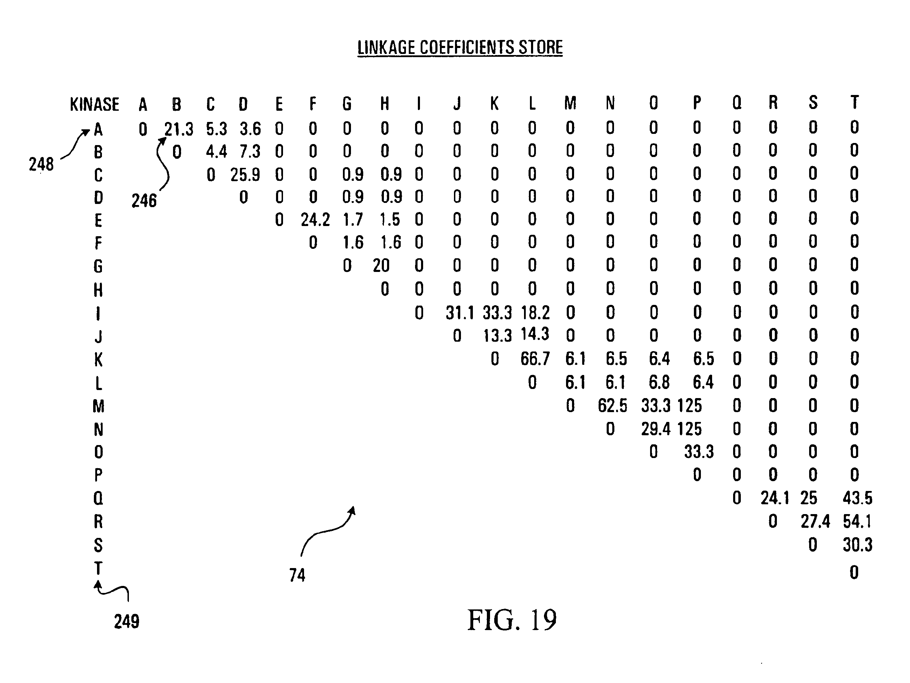

Referring to Figures **18** and **19****,** block **244** further directs the processor circuit to multiply the result of the above division by **100** to produce the linkage coefficient, and to store the linkage coefficient in the linkage coefficients store **74,** or more particularly, in a linkage coefficient field **246** of a cell signaling protein linkage record **248** corresponding to the first cell signaling protein of the corresponding pair of cell signaling proteins. As shown in Figure **19****,** each cell signaling protein linkage record **248** includes a cell signaling protein identification field **249,** and further includes a plurality of linkage coefficient fields **246,** containing a plurality of respective linkage coefficients corresponding to the linkage of the cell signaling protein identified in the identification field **249** and every other successive cell signaling protein (e.g. every kinase whose corresponding linkage record **248** appears beneath the current linkage record **248** in the linkage coefficients store **74** shown in Figure **19****).** It will be appreciated that for the purpose of analyzing linkage, it is unnecessary to treat the cell signaling protein pairs as permutations rather than combinations, as the linkage of B with A for example is necessarily identical to the linkage of A with B. However, if desired, each linkage record **248** may redundantly contain linkage coefficients for the identified cell signaling protein and all other cell signaling proteins, rather than merely for successive cell signaling proteins. In examining the contents of the linkage coefficients store **74,** it will be appreciated that a disproportionately large number of "zero" linkage coefficients are present, as a result of the decision at block **216** of the coregulation routine **42** to universally set negative coregulation coefficients equal to zero, as discussed above.

When all such linkage coefficients have been produced and stored in the linkage coefficients store **74,** block **244** further directs the processor circuit to copy the linkage coefficients store **74** to the data storage area **110** of the storage medium **22,** for future retrieval if desired.

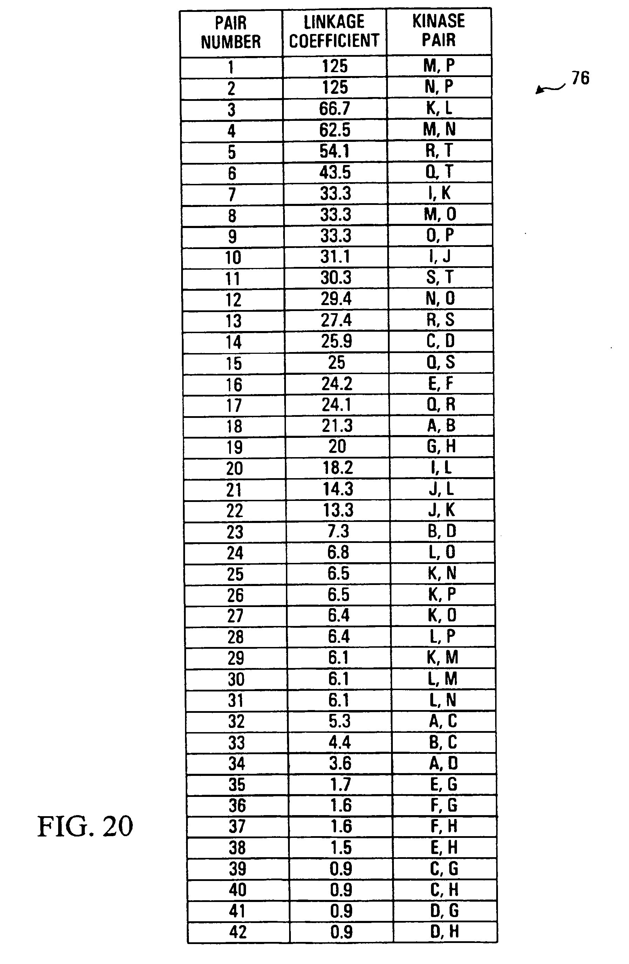

Referring to Figures **2****, 18** and **20****,** block **250** then configures the processor circuit to produce a list of linked cell signaling protein pairs, the list including an identification of each cell signaling protein pair having a linkage coefficient greater than or equal to a threshold linkage value. In this embodiment, the threshold linkage value is **0.5,** however, other threshold values may be substituted. Block **250** further directs the processor circuit to sort the list of such linked cell signaling protein pairs by linkage coefficient in descending order, and to store the sorted list as the contents of the linkage-sorted pairs store **76** shown in Figure **20****.** A copy of the linkage-sorted pairs store **76** is then copied to the data storage area **110** of the storage medium **22.**

Blocks **252** to **262** then configure the processor circuit **16** to associate at least some of the cell signaling proteins with respective common signaling pathways, in response to the linkage coefficients. More particularly, blocks **252** to **262** configure the processor circuit to associate the cell signaling proteins with the pathways by identifying a group of the cell signaling proteins for which each linkage coefficient linking each cell signaling protein to each other cell signaling protein of the group is greater than or equal to a threshold linkage value. In this manner, the processor circuit is configured to produce lists of the common signaling pathways.

Block **252** first directs the processor circuit to address a first individual cell signaling protein present in at least one pair in the linkage-sorted pairs store **76.**

Block **254** then configures the processor circuit **16** to generate a linkage list including an identification of the currently addressed cell signaling protein. Block **254** further directs the processor circuit to store the newly-generated linkage list in the linked pathway groups store **78.**

Block **256** configures the processor circuit **16** to add, to the new linkage list, an identification of each other cell signaling protein for which the linkage coefficient for the currently addressed cell signaling protein and the other cell signaling protein is greater than or equal to the threshold linkage value. For example, referring to Figures **18,** **19** and **21****,** if the currently addressed cell signaling protein is kinase C, then a new linkage list 257 corresponding to kinase C will temporarily consist of kinases A, B, C, D, G and H.

Block **258** then configures the processor circuit to eliminate, from the linkage list, each cell signaling protein on the linkage list for which the linkage coefficient for that cell signaling protein and at least one other cell signaling protein on the linkage list is less than the threshold linkage value. Continuing the previous example wherein the currently addressed cell signaling protein is kinase C, block **258** will direct the processor circuit to eliminate kinases A and B from the linkage list **257,** because each of A and B has a linkage coefficient less than the threshold linkage value for at least one other kinase in the linkage list (such as G for example), leaving only kinases C, D, G and H remaining in the new linkage list **257** stored in the linked pathway groups store **78.**

In effect, therefore, blocks **252** to **258** serve to produce a linked group of cell signaling proteins, for which every member of the group has a linkage coefficient with every other member of the group greater than or equal to the threshold linkage value.

Block **260** directs the processor circuit to determine whether all of the cell signaling proteins in the linkage-sorted pairs store **76** have been addressed to produce respective linked pathway groups in this manner. If not, block **262** directs the processor circuit to address the next such cell signaling protein, and the processor circuit is directed back to block **254** to produce a new linkage list corresponding to the newly addressed cell signaling protein, and to store the new linkage list in the linked pathway groups store **78.**

If at block **260,** linkage lists representing respective linked pathway groups have been produced for all cell signaling proteins, block **264** directs the processor circuit **16** to compare the various linkage lists stored in the linked pathway groups store **78** to each other, and to delete any redundant duplicate linkage lists that may exist therein. Block **264** then directs the processor circuit to copy the linked pathway groups store **78** to the data storage area **110** of the storage medium **22,** for future retrieval if desired.

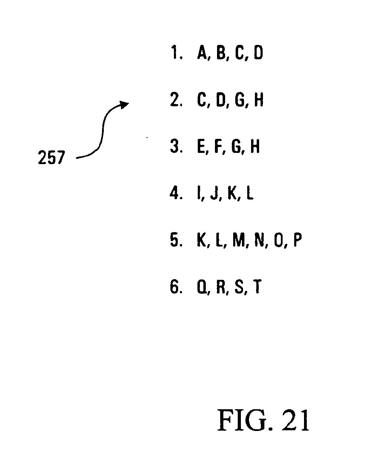

Block **266** then directs the processor circuit to output the contents of the linkage-sorted pairs store **76** and the linked pathway groups store **78.** The output of the contents of the linked pathway groups store **78** directly represents groups of cell signaling proteins forming respective common signaling pathways, such as the six signaling pathway groups of kinases shown in Figure **21****.** As with the coexpression and coregulation outputs discussed above, such outputs may include printouts, recordings on media, transmissions, or other forms of output.

The linkage routine **44** is then ended.

### Further Measurements

Once a number of common signaling pathways have been identified, such as the groups listed in the linked pathway groups store **78** for example, the next desirable step is to determine the "order" of the cell signaling proteins within each signaling pathway. Only limited inferences in this regard may be drawn from the foregoing linkage coefficient analysis.

However, by performing additional time course studies, in which the phosphorylation states of cell signaling proteins such as kinases and their substrates are carefully monitored at various times immediately after the exposure of a cell to different stimuli, it may be possible to determine the order of the cell signaling proteins within each signaling pathway. For example, in each data set, a time after the introduction of a stimulus at which a given kinase has experienced a **50**% of maximal change in phosphorylation may be recorded, and the average such time over all data sets may be calculated. Such average phosphorylation change times may then be employed to determine a signaling order within a given signaling pathway. It may then be possible to derive a suitable set of rules for determining, for example, whether a given upstream kinase within a signaling pathway is triggering two subsequent downstream kinases in series or in parallel.

While specific embodiments of the invention have been described and illustrated, such embodiments should be considered illustrative of the invention only and not as limiting the invention as construed in accordance with the accompanying claims.

## Claims

1. A method of analysing cell signaling proteins being detected in a single biological sample so as to identify cell signaling proteins that are associated with one another in common signaling pathways, the method comprising the steps of
a. producing in suitable hardware means (15,300,302) data values representing the physical properties of the detected cell proteins;
b. receiving in a receiver (12) said data values representing physical properties of the detected cell signaling proteins;
c. providing a processor circuit (16) comprising a memory (18), an analysing process to be executed by the processor circuit, and configuring means (coexpression routine 40; coregulation routine 42; linkage routine 44; clustering subroutine 46) adapted to direct the processor circuit (16) to execute the analysing process;
d. storing in said memory (18) for each detected cell signaling protein the received cell signaling protein data values in addressable data records;
e. executing the analysing process in the processor circuit (16) so as to produce and store, in the memory (18), a comparison value for each possible pair of detected cell signaling proteins in response to the received cell signaling protein data values, said comparison value being a coefficient representing a degree of association between one cell signaling protein of the pair and the other cell signaling protein of the pair;
f. identifying cell signaling protein pairs having comparison values satisfying a condition indicative of an association between the cell proteins; and
producing, and storing in the memory, a list of identifications of pairs of associated cell signaling proteins.

2. A method as claimed in claim 1, in which the analysing process comprises a coexpression routine.

3. A method as claimed in claim 1 in which the analysing process comprises a coregulation routine.

4. A method as claimed in claim 1, further comprising input means for user-selecting an analysing process from a plurality of predetermined analysing processes, the method comprising selecting said analysing process from said plurality of analysing processes, wherein said configuring means comprises
means (40) adapted to configure the processor circuit to produce, as the comparison values, a coexpression coefficient for each pair of the cell signaling proteins, each coexpression coefficient representing a degree of coexpression of one cell signaling protein of the pair and the other cell protein of the pair;
means (42) adapted to configure the processor circuit to produce, as the comparison values, a coregulation coefficient for each pair of the cell signaling proteins, each coregulation coefficient representing a degree of coregulation of one cell signaling protein of the pair and the other cell signaling protein of the pair; and
means (42) adapted to configure the processor circuit to produce, as the comparison value, a linkage coefficient, consisting of the coregulation coefficient divided by the coexpression coefficient, for each pair of the cell signaling proteins, each linkage coefficient representing a degree of association of one cell signaling protein of the pair and the other cell signaling protein of the pair.

5. The method of any preceding claim wherein storing comprises storing in a random access memory, said comparison values.

6. The method of any preceding claim further comprising normalizing said data values relative to at least one reference value, prior to producing said comparison values.

7. The method of claim 1 wherein identifying comprises producing a list of clusters of associated cell signaling proteins.

8. The method of any preceding claim wherein identifying further comprises identifying a cluster of associated cell signaling proteins, said cluster comprising a group of said pairs of associated cell signaling proteins for which each member of each said pair is present in at least one other pair of said group.

9. The method of claim 1 wherein identifying each said cluster comprises:
a) generating a cluster list associated with a first cell signaling protein pair, said cluster list comprising an identification of said first cell signaling protein pair;
b) adding, to said cluster list, an identification of each of said pairs that includes at least one cell signaling protein already present in said cluster list;
c) repeating said adding following each said adding of pairs to said cluster list, to effectively add to said cluster list each of said pairs that includes at least one cell signaling protein present in at least one pair added to said cluster list;
d) eliminating, from said cluster list, each of said pairs that includes at least one cell signaling protein not found in at least one other pair in said cluster list; and
e) repeating said eliminating following each said eliminating of pairs, to effectively eliminate from said cluster list each of said pairs that includes at least one cell signaling protein not present in at least one other non-eliminated pair in said cluster list.

10. The method of claim 2 or 4 wherein the analysing process producing each said coexpression coefficient comprises:
a) for each set, calculating a difference value equal to an absolute value of a difference between said data value corresponding to said one cell signaling protein and said data value corresponding to said other cell signaling protein; and
b) adding said difference values for each of said sets to produce a sum of difference values.

11. The method of claim **10** wherein the analysing process producing each said coexpression coefficient further comprises dividing said sum by the number of said sets to produce said coexpression coefficient corresponding to said one cell signaling protein and said other cell signaling protein.

12. The method of claim **10** wherein identifying cell signaling protein pairs comprises identifying each cell signaling protein pair having a coexpression coefficient less than or equal to a threshold coexpression value.

13. The method of claim **10** wherein identifying cell signaling protein pairs comprises producing a list of coexpressed cell signaling protein pairs, said list comprising an identification of each said cell signaling protein pair having a coexpression coefficient less than or equal to said threshold coexpression value.

14. The method of claim **13** wherein identifying cell signaling protein pairs further comprises producing a list of clusters of coexpressed cell signaling protein pairs, each said cluster comprising a group of said coexpressed cell signaling protein pairs for which each member of each pair is present in at least one other pair of said group.

15. The method of claim **1** wherein each set of data values indicates phosphorylation states of respective cell, signaling proteins in biological material corresponding to said set.

16. The method of claim **15** wherein producing said comparison values comprises producing a coregulation coefficient for each pair of said cell signaling proteins, each said coregulation coefficient representing a degree of coregulation of one cell signaling protein of said pair and the other cell signaling protein of said pair.

17. The method of claim **16** wherein producing each said coregulation coefficient comprises:
a) for each set, assigning a pair state value as a function of phosphorylation states of said one cell signaling protein and said other cell signaling protein; and
b) adding said pair state values for each of said sets to produce a sum of pair state values.

18. The method of claim **17** wherein producing each said coregulation coefficient further comprises dividing said sum by the number of said sets to produce said coregulation coefficient corresponding to said one cell signaling protein and said other cell signaling protein.

19. The method of claim **17** wherein assigning a pair state value comprises:
a) assigning a first pair state value when said one cell signaling protein and said other cell signaling protein are both in a phosphorylated state;
b) assigning a second pair state value when said one cell signaling protein and said other cell signaling protein are both in a dephosphorylated state, said second pair state value being less than said first pair state value; and
c) assigning a third pair state value when said one cell signaling protein and said other cell signaling protein are in different phosphorylation states, said third pair state value being less than said second pair state value.

20. The method of claim **17** wherein identifying cell signaling protein pairs comprises identifying each cell signaling, protein pair having a coregulation coefficient greater than a threshold coregulation value.

21. The method of claim **20** wherein identifying cell signaling protein pairs further comprises producing a list of coregulated cell signaling protein pairs, said list comprising an identification of each said cell signaling protein pair having a coregulation coefficient greater than said threshold coregulation value.

22. The method of claim **21** wherein identifying cell signaling protein pairs further comprises producing a list of clusters of coregulated cell signaling protein pairs, each said cluster comprising a group of said coregulated cell signaling protein pairs for which each member of each pair is present in at least one other pair of said group.

23. The method of claim **1** wherein producing a comparison value comprises producing a linkage coefficient for each pair of said cell signaling proteins, as a function of a coexpression coefficient representing a degree of coexpression of one cell signaling protein of said pair and the other cell signaling protein of said pair, and of a coregulation coefficient representing a degree of coregulation of said one cell signaling protein of said pair and said other cell signaling protein of said pair, each said linkage coefficient representing a degree of association between said one cell signaling protein and said other cell signaling protein.

24. The method of claim **23** wherein producing said linkage coefficient comprises, for each said pair, dividing said coregulation coefficient by said coexpression coefficient.

25. The method of claim **24** further comprising producing a list of linked cell signaling protein pairs, said list comprising an identification of each said cell signaling protein pair having a linkage coefficient greater than or equal to a threshold linkage value.

26. The method of claim **24** further comprising associating at least some of said cell signaling proteins with respective common signaling pathways, in response to said linkage coefficients.

27. The method of claim **26** wherein associating comprises identifying a group of said cell signaling proteins for which each said linkage coefficient linking each cell signaling protein to each other cell signaling protein of said group is greater than or equal to a threshold linkage value.

28. The method of claim **27** wherein identifying said group comprises:
a) generating a linkage list comprising a first cell signaling protein:
b) adding, to said linkage list, each other said cell signaling protein for which said linkage coefficient for said first cell signaling protein and said other cell signaling protein is greater than or equal to said threshold linkage value; and
c) eliminating, from said linkage list, each cell signaling protein on said linkage list for which said linkage coefficient for said each cell signaling protein and at least one other cell signaling protein on said linkage list is less than said threshold linkage value.

29. The method of claim **27** further comprising producing lists of said common signaling pathways.

30. The method of claim **29** wherein said data values are representative of proteins in a single dimension in an electrophoresis gel.

31. The method of claim **30** further comprising producing said electrophoresis gel.

32. An apparatus being adapted to identify associated cell signaling proteins according to the method of claim 1, the apparatus comprising:
a) a receiver operable to receive data values representing physical properties of respective cell signaling proteins; and
b) a processor circuit (16) in communication with said receiver, said processor circuit comprising a memory (18) said processor circuit (16) being configured to execute an analysing process to:
i) produce and store, in the memory (18), a comparison value for each pair of said cell signaling proteins in response to said data values; and
ii) Identify cell signaling protein pairs having comparison values satisfying a condition indicative of an association between the cell signaling proteins; and produce and store in the memory (18) a list of identifications of pairs of associated cell signalling proteins.

33. An apparatus as claimed in claim 32, further comprising input means for user-selecting said analysing process from a plurality of predetermined analysing processes, wherein said configuring means comprises
means (40) adapted to configure the processor circuit to produce, as the comparison values, a coexpression coefficient for each pair of the cell signaling proteins, each coexpression coefficient representing a degree of coexpression of one cell signaling protein of the pair and the other cell signaling protein of the pair;
means (42) adapted to configure the processor circuit to produce, as the comparison values, a coregulation coefficient for each pair of the cell signaling proteins, each coregulation coefficient representing a degree of coregulation of one cell signaling protein of the pair and the other cell signaling protein of the pair; and
means (42) adapted to configure the processor circuit to produce, as the comparison value, a linkage coefficient, consisting of the coregulation coefficient divided by the coexpression coefficient, for each pair of the cell signaling proteins, each linkage coefficient representing a degree of association of one cell signaling protein of the pair and the other cell signaling protein of the pair.

34. The apparatus of claim **33** wherein said memory comprises a random access memory.

35. The apparatus of claim **34** wherein said processor circuit is configured to normalize said data values relative to at least one reference value, prior to producing said comparison values.

36. The apparatus of claim **34** wherein said processor circuit is configured to produce a list of pairs of associated cell signaling proteins.

37. The apparatus of claim **34** wherein said processor circuit is configured to produce a list of clusters of associated cell signaling proteins.

38. The apparatus of claim **36** wherein said processor circuit is configured to identify a cluster of associated cell signaling proteins, said cluster comprising a group of said pairs of associated cell signaling proteins for which each member of each said pair is present in at least one other pair of said group.

39. The apparatus of claim **38** wherein said processor circuit is configured to identify said cluster of associated cell signaling proteins, by:
a) generating a cluster list associated with a first cell signaling protein pair, said cluster list comprising an identification of said first cell signaling protein pair;
b) adding, to said cluster list, an identification of each of said pairs that includes at least one cell signaling protein already present in said cluster list;
c) repeating said adding following each said adding of pairs to said cluster list, to effectively add to said cluster list each of said pairs that includes at least one cell signaling protein present in at least one pair added to said cluster list;
d) eliminating, from said cluster list, each of said pairs that includes at least one cell signaling protein not found in at least one other pair in said cluster list; and
e) repeating said eliminating following each said eliminating of pairs, to effectively eliminate from said cluster list each of said pairs that includes at least one cell signaling protein not present in at least one other non-eliminated pair in said cluster list.

40. The apparatus of claim **32** wherein said receiver is operable to receive, sets of cell signaling protein data, each set comprising said data values, said data values representing amounts of respective corresponding cell signaling proteins in biological material corresponding to said set.

41. The apparatus of claim **40** wherein said processor circuit is configured to produce, as said comparison values, a coexpression coefficient for each pair of said cell signaling proteins, each said coexpression coefficient representing a degree of coexpression of one cell signaling protein of said pair and the other cell signaling protein of said pair.

42. The apparatus of claim **41** wherein said processor circuit is configured to produce each said coexpression coefficient by:
a) for each set, calculating a difference value equal to an absolute value of a difference between said data value corresponding to said one cell signaling protein and said data value corresponding to said other cell signaling protein; and
b) adding said difference values for each of said sets to produce a sum of difference values.

43. The apparatus of claim **42** wherein said processor circuit is configured to divide said sum by the number of said sets to produce said coexpression coefficient corresponding to said one cell signaling protein and said other cell signaling protein.

44. The apparatus of claim **42** wherein said processor circuit is configured to identify said cell signaling protein pairs by identifying each cell signaling protein pair having a coexpression coefficient less than or equal to a threshold coexpression value.

45. The apparatus of claim **42** wherein said processor circuit is configured to produce a list of coexpressed cell signaling protein pairs, said list comprising an identification of each said cell signaling protein pair having a coexpression coefficient less than or equal to said threshold coexpression value.

46. The apparatus of claim **45** wherein said processor circuit is configured to produce a list of clusters of coexpressed cell signaling protein pairs, each said cluster comprising a group of said pairs of coexpressed cell signaling proteins for which each member of each pair is present in at least one other pair of said group.

47. The apparatus of claim **32** wherein said receiver is operable to receive sets of cell signaling protein data, each set comprising said data values, said data values indicating phosphorylation states of respective cell signaling proteins in biological material corresponding to said set.

48. The apparatus of claim **47** wherein said processor circuit is configured to produce, as said comparison values, a coregulation coefficient for each pair of said cell signaling proteins, each said coregulation coefficient representing a degree of coregulation of one cell signaling protein of said pair and the other cell signaling protein of said pair.

49. The apparatus of claim **48** wherein said processor circuit is configured to produce each said coregulation coefficient by:
a) for each set, assigning a pair state value as a function of phosphorylation states of said one cell, signaling protein and said other cell signaling protein; and
b) adding said pair state values for each of said sets to produce a sum of pair state values.

50. The apparatus of claim **49** wherein said processor circuit is configured to divide said sum by the number of said sets to produce said coregulation coefficient corresponding to said one cell signaling protein and said other cell signaling protein.

51. The apparatus of claim **49** wherein wherein said processor circuit is configured to assign said pair state value by:
a) assigning a first pair state value when said one cell signaling protein and said other cell signaling protein are both in a phosphorylated state;
b) assigning a second pair state value when said one cell signaling protein and said other cell signaling protein are both in a dephosphorylated state, said second pair state value being less than said first pair state value; and
c) assigning a third pair state value when said one cell signaling protein and said other cell signaling protein are in different phosphorylation states, said third pair state value being less than said second pair state value.

52. The apparatus of claim **49** wherein said processor circuit is configured to identify said cell signaling protein pairs by identifying each cell signaling protein pair having a coregulation coefficient greater than a threshold coregulation value.

53. The apparatus of claim **52** wherein said processor circuit is configured to produce a list of coregulated cell signaling protein pairs, said list comprising an identification of each said cell signaling protein pair having a coregulation coefficient greater than said threshold coregulation value.

54. The apparatus of claim **53** wherein said processor circuit is configured to produce a list of clusters of coregulated cell signaling protein pairs, each said cluster comprising a group of said coregulated cell signaling protein pairs for which each member of each pair is present in at least one other pair of said group.

55. The apparatus of claim **32** wherein said processor circuit is configured to produce, as said comparison values, a linkage coefficient for each pair of said cell signaling proteins, as a function of a coexpression coefficient representing a degree of coexpression of one cell signaling protein of said pair and the other cell signaling protein of said pair, and of a coregulation coefficient representing a degree of coregulation of said one cell signaling protein of said pair and said other cell signaling protein of said pair, each said linkage coefficient representing a degree of association between said one cell signaling protein and said other cell signaling protein.

56. The apparatus of claim **55** wherein said processor circuit is configured to produce each said linkage coefficient by, for each said pair, dividing said coregulation coefficient by said coexpression coefficient.

57. The apparatus of claim **56** wherein said processor circuit is configured to produce a list of linked cell signaling protein pairs, said list, comprising an identification of each said cell signaling protein pair having a linkage coefficient greater than or equal to a threshold linkage value.

58. The apparatus of claim **56** wherein said processor circuit is configured to associate at least some of said cell signaling proteins with respective common signaling pathways, in response to said linkage coefficients.

59. The apparatus of claim **58** wherein said processor circuit is configured to associate said cell signaling proteins with said pathways by identifying a group of said cell signaling proteins for which each said linkage coefficient linking each cell signaling protein to each other cell signaling protein of said group is greater than or equal to a threshold linkage value.

60. The apparatus of claim **59** wherein said processor circuit is configured to identify said group by:
a) generating a linkage list comprising a first cell signaling protein;
b) adding, to said linkage list, each other said cell, signaling protein for which said linkage coefficient for said first cell signaling protein and said other cell signaling protein is greater than or equal to said threshold linkage value; and
c) eliminating, from said linkage list, each cell signaling protein on said linkage list for which said linkage coefficient for said each cell signaling protein and at least one other cell signaling protein on said linkage list is less than said threshold linkage value.

61. The apparatus of claim **59** wherein said processor circuit is configured to produce lists of said common signaling pathways.

62. The apparatus of claim **32** further comprising a measuring device operable to produce said data values representing said physical properties of said respective cell signaling proteins.

63. The apparatus of claim **62** wherein said measuring device comprises a chemiluminescence imager operable to produce signals representative of proteins in a single dimension in an electrophoresis gel.

64. The apparatus of claim **63** wherein said measuring device further comprises an electrophoresis apparatus operable to produce said electrophoresis gel.

65. The apparatus of claim **64** wherein said electrophoresis apparatus comprises a one-dimensional sodium dodecyl sulphate polyacrylamide gel electrophoresis (SDS-PAGE) measuring system.

66. A computer readable medium for providing instructions for directing a programmable device to execute the method of claim **1**.

67. A computer data signal embodied in a carrier wave, the signal comprising code segments for directing a programmable device to execute the method of claim **1**.

68. A computer program comprising code means that when executed by a processor circuit carries out all the steps of the method of claim **1**.

## Patentansprüche

1. Verfahren zur Analyse von Zellsignalproteinen, die in einer einzelnen biologischen Probe nachgewiesen werden, um Zellsignalproteine zu identifizieren, die miteinander in gemeinsamen Signalwegen assoziiert sind, wobei das Verfahren die folgenden Schritte umfasst:
a. Erstellung von Datenwerten, die die physikalischen Eigenschaften der nachgewiesenen Zellsignalproteine darstellen, in geeigneten Hardware-Mitteln (15,300,302);
b. Empfang dieser Datenwerte, die die physikalischen Eigenschaften der nachgewiesenen Zellsignalproteine darstellen, in einem Empfänger (12);
c. Zur-Verfügung-Stellung einer Prozessorschaltung (16), umfassend einen Speicher (18), einen Analyseprozess zur Ausführung durch die Prozessorschaltung, und Konfigurationsmittel (Coexpressionsroutine 40; Coregulationsroutine 42; Verlinkungsroutine 44; Clustering-Subroutine 46), die dafür adaptiert sind, die Prozessorschaltung (16) anzusteuern, den Analyseprozess durchzuführen;
d. Abspeichern der erhaltenen Datenwerte der Zellsignalproteine für jedes nachgewiesene Zellsignalprotein im Speicher (18) in adressierbaren Datenaufzeichnungen;
e. Durchführung des Analyseprozesses in der Prozessorschaltung (16), um einen Vergleichswert für jedes mögliche Paar nachgewiesener Zellsignalproteine als Antwort auf die erhaltenen Datenwerte der Zellsignalproteine zu erstellen und im Speicher (18) abzulegen, wobei es sich bei diesem Vergleichswert um einen Koeffizienten handelt, der einen Grad der Assoziation zwischen dem einen Zellsignalprotein des Paares und dem anderen Zellsignalprotein des Paares darstellt;
f. Identifikation von Zellsignalprotein-Paaren mit Vergleichswerten, die eine Bedingung erfüllen, die ein Indikator für eine Assoziation zwischen den Zellsignalproteinen ist; und
g. Erstellung einer Liste von Identifikationen von Paaren assoziierter Zellsignalproteine und Ablegen im Speicher.

2. Verfahren gemäß Anspruch 1, worin der Analyseprozess eine Coexpressionsroutine umfasst.

3. Verfahren gemäß Anspruch 1, worin der Analyseprozess eine Coregulationsroutine umfasst.

4. Verfahren gemäß Anspruch 1, weiters umfassend Eingabemittel für die Auswahl eines Analyseprozesses aus einer Vielzahl von vorbestimmten Analyseprozessen durch den Benutzer, wobei das Verfahren die Auswahl des Analyseprozesses aus einer Vielzahl von Analyseprozessen umfasst, wobei das Konfigurationsmittel folgendes umfasst:
Mittel (40), die adaptiert sind, um die Prozessorschaltung zu konfigurieren, um als Vergleichswerte einen Coexpressions-Koeffizienten für jedes Paar der Zellsignalproteine zu erstellen, wobei jeder Coexpressions-Koeffizient einen Grad der Coexpression des einen Zellsignalproteins des Paares und des anderen Zellsignalproteins des Paares darstellt;
Mittel (42), die adaptiert sind, um die Prozessorschaltung zu konfigurieren, um als Vergleichswerte einen Coregulations-Koeffizienten für jedes Paar der Zellsignalproteine zu erstellen, wobei jeder Coregulations-Koeffizient einen Grad der Coregulation des einen Zellsignalproteins des Paares und des anderen Zellsignalproteins des Paares darstellt; und
Mittel (42), die adaptiert sind, um die Prozessorschaltung zu konfigurieren, um als Vergleichswert für jedes Paar der Zellsignalproteine einen Verlinkungs-Koeffizienten, bestehend aus dem Coregulations-Koeffizienten dividiert durch den Coexpressions-Koeffizienten, zu erstellen, wobei jeder Verlinkungs-Koeffizient einen Grad der Assoziation des einen Zellsignalproteins des Paares und des anderen Zellsignalproteins des Paares darstellt.

5. Verfahren gemäß einem der vorstehenden Ansprüche, worin das Ablegen das Abspeichern dieser Vergleichswerte in einem Random Access-Speicher umfasst.

6. Verfahren gemäß einem der vorstehenden Ansprüche, weiters umfassend die Normalisierung dieser Datenwerte in Bezug auf mindestens einen Referenzwert vor der Erstellung der Vergleichswerte.

7. Verfahren gemäß Anspruch 1, worin die Identifikation die Erstellung einer Liste von Clustern assoziierter Zellsignalproteine umfasst.

8. Verfahren gemäß einem der vorstehenden Ansprüche, worin die Identifikation weiters die Identifikation eines Clusters assoziierter Zellsignalproteine umfasst, wobei dieser Cluster eine Gruppe der Paare assoziierter Zellsignalproteine umfasst, für welche jedes Mitglied jedes Paares in mindestens einem anderen Paar dieser Gruppe vorhanden ist.

9. Verfahren gemäß Anspruch 1, worin die Identifikation jedes Clusters folgendes umfasst:
a) Erstellung einer Clusterliste, die mit einem ersten Zellsignalprotein-Paar assoziiert ist, wobei diese Clusterliste eine Identifikation des ersten Zellsignalprotein-Paares umfasst;
b) Hinzufügung einer Identifikation jedes dieser Paare, das zumindest ein Zellsignalprotein enthält, das auf dieser Clusterliste bereits vorhanden ist, zu dieser Clusterliste;
c) Wiederholung dieser Hinzufügung nach jeder Hinzufügung von Paaren zu der Clusterliste, um zu der Clusterliste effektiv jedes dieser Paare hinzuzufügen, das zumindest ein Zellsignalprotein enthält, das zumindest in einem Paar, das zu der Clusterliste hinzugefügt wurde, vorhanden ist;
d) Streichung jedes Paares, das zumindest ein Zellsignalprotein beinhaltet, das nicht in mindestens einem anderen Paar auf der Clusterliste zu finden ist, aus dieser Clusterliste; und
e) Wiederholen dieser Streichung nach jeder Streichung von Paaren, um effektiv jedes Paar, das mindestens ein Zellsignalprotein enthält, das nicht in mindestens einem anderen, nicht gestrichenen Paare auf der Clusterliste vorhanden ist, aus dieser Clusterliste zu streichen.

10. Verfahren gemäß Anspruch 2 oder 4, worin der Analyseprozess zur Erstellung des Coexpressions-Koeffizienten folgendes umfasst:
a) Für jedes Set die Berechnung des Differenzwertes gleich einem absoluten Wert einer Differenz zwischen dem Datenwert entsprechend dem einen Zellsignalprotein und dem Datenwert entsprechend dem anderen Zellsignalprotein; und
b) Addition dieser Differenzwerte für jedes Set zum Erhalt einer Summe von Differenzwerten.

11. Verfahren gemäß Anspruch 10, worin der Analyseprozess zur Erstellung jedes Coexpressions-Koeffizienten weiters das Dividieren dieser Summe durch die Anzahl der Sets umfasst, um den Coexpressions-Koeffizienten zu erhalten, der dem einen Zellsignalprotein und dem anderen Zellsignalprotein entspricht.

12. Verfahren gemäß Anspruch 10, worin die Identifikation von Zellsignalprotein-Paaren die Identifikation jedes Zellsignalprotein-Paares umfasst, das einen Coexpressions-Koeffizienten kleiner oder gleich einem Coexpressions-Schwellenwert aufweist.

13. Verfahren gemäß Anspruch 10, worin die Identifikation von Zellsignalprotein-Paaren die Erstellung einer Liste von coexprimierten Zellsignalprotein-Paaren umfasst, wobei die Liste eine Identifikation jedes Zellsignalprotein-Paares umfasst, das einen Coexpressions-Koeffizienten aufweist, der kleiner oder gleich dem Coexpressions-Schwellenwert ist.

14. Verfahren gemäß Anspruch 13, worin die Identifikation von Zellsignalprotein-Paaren weiters die Erstellung einer Liste von Clustern von coexprimierten Zellsignalproteinen umfasst, wobei jedes dieser Cluster eine Gruppe der coexprimierten Zellsignalprotein-Paare umfasst, für welches jedes Mitglied jedes Paares in mindestens einem anderen Paar der Gruppe vorhanden ist.

15. Verfahren gemäß Anspruch 1, worin jedes Set von Datenwerten die Phosphorylierungszustände der jeweiligen Zellsignalproteine in biologischem Material entsprechend dem Set angibt.

16. Verfahren gemäß Anspruch 15, worin die Erstellung der Vergleichswerte die Erstellung eines Coregulations-Koeffizienten für jedes Paar der Zellsignalproteine umfasst, wobei der Coregulations-Koeffizient einen Grad der Coregulation des einen Zellsignalproteins des Paares und des anderen Zellsignalproteins des Paares darstellt.

17. Verfahren gemäß Anspruch 16, worin die Erstellung jedes Coregulations-Koeffizienten umfasst:
a) für jedes Set die Zuordnung eines Paar-Zustands-Werts als eine Funktion der Phosphorylierungszustände des einen Zellsignalproteins und des anderen Zellsignalproteins; und
b) die Addition des Paar-Zustands-Werts für jedes Set zur Erstellung einer Summe von Paar-Zustands-Werten.

18. Verfahren gemäß Anspruch 17, worin die Erstellung jedes Coregulations-Koeffizienten weiters die Division der Summe durch die Anzahl der Sets umfasst, um den Coregulations-Koeffizienten entsprechend dem einen Zellsignalprotein und dem anderen Zellsignalprotein zu ergeben.

19. Verfahren gemäß Anspruch 17, worin die Zuordnung eines Paar-Zustands-Werts folgendes umfasst:
a) die Zuordnung eines ersten Paar-Zustands-Werts, wenn sich sowohl das eine Zellsignalprotein als auch das andere Zellsignalprotein in einem phosphorylierten Zustand befinden;
b) die Zuordnung eines zweiten Paar-Zustands-Werts, wenn sich sowohl das eine Zellsignalprotein als auch das andere Zellsignalprotein in einem dephosphorylierten Zustand befinden, wobei der zweite Paar-Zustands-Wert geringer ist als der erste Paar-Zustands-Wert; und
c) die Zuordnung eines dritten Paar-Zustands-Werts, wenn sich das eine Zellsignalprotein und das andere Zellsignalprotein in verschiedenen Phosphorylierungszuständen befinden, wobei der dritte Paar-Zustands-Wert geringer ist als der zweite Paar-Zustands-Wert.

20. Verfahren gemäß Anspruch 17, worin die Identifikation von Zellsignalprotein-Paaren die Identifikation jedes Zellsignalproteins-Paares mit einem Coregulations-Koeffizienten umfasst, der größer ist als ein Coregulations-Schwellenwert.

21. Verfahren gemäß Anspruch 20, worin die Identifikation von Zellsignalprotein-Paaren weiters die Erstellung einer Liste von coregulierten Zellsignalprotein-Paaren umfasst, wobei die Liste eine Identifikation jedes Zellsignalprotein-Paares umfasst, dessen Coregulations-Koeffizient größer ist als der Coregulations-Schwellenwert.

22. Verfahren gemäß Anspruch 21, worin die Identifikation von Zellsignalprotein-Paaren weiters die Erstellung einer Liste von Clustern coregulierter Zellsignalprotein-Paare umfasst, wobei jedes Cluster eine Gruppe von coregulierten Zellsignalprotein-Paaren umfasst, für welche jedes Mitglied jedes Paares in mindestens einem anderen Paar der Gruppe vorhanden ist.

23. Verfahren gemäß Anspruch 1, worin die Erstellung eines Vergleichswertes die Erstellung eines Verlinkungs-Koeffizienten für jedes Paar der Zellsignalproteine als Funktion eines Coexpressions-Koeffizienten, der einen Grad der Coexpression des einen Zellsignalproteins des Paares und des anderen Zellsignalproteins des Paares darstellt, und eines Coregulations-Koeffizienten umfasst, der einen Grad der Coregulation des einen Zellsignalproteins des Paares und des anderen Zellsignalproteins des Paares darstellt, wobei jeder Verlinkungs-Koeffizient einen Grad der Assoziation zwischen dem einen Zellsignalprotein und dem anderen Zellsignalprotein darstellt.

24. Verfahren gemäß Anspruch 23, worin die Erstellung des Verlinkungs-Koeffizienten für jedes Paar die Division des Coregulations-Koeffizienten durch den Coexpressions-Koeffizienten umfasst.

25. Verfahren gemäß Anspruch 24, weiters umfassend die Erstellung einer Liste von verlinkten Zellsignalprotein-Paaren, wobei diese Liste eine Identifikation jedes Zellsignalprotein-Paares mit einem Verlinkungs-Koeffizienten größer oder gleich einem Verlinkungs-Schwellenwert umfasst.

26. Verfahren gemäß Anspruch 24, weiters umfassend die Assoziation von mindestens einigen Zellsignalproteinen mit den jeweiligen gemeinsamen Signalwegen als Antwort auf die Verlinkungs-Koeffizienten.

27. Verfahren gemäß Anspruch 26, worin die Assoziation die Identifikation einer Gruppe der Zellsignalproteine umfasst, für welche jeder Verlinkungs-Koeffizient, der jedes Zellsignalprotein mit jedem anderen Zellsignalprotein der Gruppe verlinkt, größer oder gleich einem Verlinkungs-Schwellenwert ist.

28. Verfahren gemäß Anspruch 27, worin die Identifikation der Gruppe folgendes umfasst:
a) Erstellung einer Verlinkungsliste, die ein erstes Zellsignalprotein umfasst;
b) Hinzufügung jedes anderen Zellsignalproteins zu dieser Verlinkungsliste, für welches der Verlinkungs-Koeffizient für das erste Zellsignalprotein und das andere Zellsignalprotein größer oder gleich dem Verlinkungs-Schwellenwert ist; und
c) Streichung jedes Zellsignalproteins auf der Verlinkungsliste aus der Verlinkungsliste, für welches der Verlinkungs-Koeffizient für jedes Zellsignalprotein und mindestens ein anderes Zellsignalprotein auf der Verlinkungsliste kleiner ist als der Verlinkungs-Schwellenwert.

29. Verfahren gemäß Anspruch 27, weiters umfassend die Erstellung von Listen der gemeinsamen Signalwege.

30. Verfahren gemäß Anspruch 29, worin die Datenwerte repräsentativ für Proteine in einer einzigen Dimension in einem Elektrophorese-Gel sind.

31. Verfahren gemäß Anspruch 30, weiters umfassend die Herstellung des Elektrophorese-Gels.

32. Vorrichtung, adaptiert zur Identifikation assoziierter Zellsignalproteine gemäß dem Verfahren von Anspruch 1, wobei die Vorrichtung folgendes umfasst:
a) einen Empfänger, der betätigbar ist, um Datenwerte zu empfangen, die die physikalischen Eigenschaften der jeweiligen Zellsignalproteine darstellen; und
b) eine Prozessorschaltung (16) in Kommunikation mit dem Empfänger, wobei die Prozessorschaltung (16), umfassend einen Speicher (18), konfiguriert ist, um einen Analyseprozess durchzuführen, um
i) im Speicher (18) einen Vergleichswert für jedes Paar der Zellsignalproteine in Antwort auf die Datenwerte zu erstellen und zu speichern;
ii) Zellsignalprotein-Paare zu identifizieren, die Vergleichswerte aufweisen, die eine Bedingung erfüllen, die ein Indikator für eine Assoziation zwischen den Zellsignalproteinen ist; und
iii) im Speicher (18) eine Liste von Identifikationen von Paaren assoziierter Zellsignalproteine zu erstellen und zu speichern.

33. Vorrichtung gemäß Anspruch 32, weiters umfassend Eingabemittel für die Auswahl des Analyseprozesses aus einer Vielzahl vorbestimmter Analyseprozesse durch den Benutzer, wobei das Konfigurierungsmittel folgendes umfasst:
Mittel (40), die adaptiert sind, um die Prozessorschaltung zu konfigurieren, um als Vergleichswerte einen Coexpressions-Koeffizienten für jedes Paar der Zellsignalproteine zu erstellen, wobei jeder Coexpressions-Koeffizient einen Grad der Coexpression des einen Zellsignalproteins des Paares und des anderen Zellsignalproteins des Paares darstellt;
Mittel (42), die adaptiert sind, um die Prozessorschaltung zu konfigurieren, um als Vergleichswerte einen Coregulations-Koeffizienten für jedes Paar der Zellsignalproteine zu erstellen, wobei jeder Coregulations-Koeffizient einen Grad der Coregulation des einen Zellsignalproteins des Paares und des anderen Zellsignalproteins des Paares darstellt; und
Mittel (42), die adaptiert sind, um die Prozessorschaltung zu konfigurieren, um als Vergleichswert einen Verlinkungs-Koeffizienten für jedes Paar der Zellsignalproteine zu erstellen, wobei der Verlinkungs-Koeffizient aus dem Coregulations-Koeffizienten dividiert durch den Coexpressions-Koeffizienten besteht und jeder Verlinkungs-Koeffizient einen Grad der Assoziation des einen Zellsignalproteins des Paares und des anderen Zellsignalproteins des Paares darstellt.

34. Vorrichtung gemäß Anspruch 33, worin der Speicher ein Random Access-Speicher umfasst.

35. Vorrichtung gemäß Anspruch 34, worin die Prozessorschaltung konfiguriert ist, die Datenwerte vor der Erstellung der Vergleichswerte in Bezug auf mindestens einen Referenzwert zu normalisieren.

36. Vorrichtung gemäß Anspruch 34, worin die Prozessorschaltung konfiguriert ist, eine Liste von Paaren assoziierter Zellsignalproteine zu erstellen.

37. Vorrichtung gemäß Anspruch 34, worin die Prozessorschaltung konfiguriert ist, eine Liste von Clustern assoziierter Zellsignalproteine zu erstellen.

38. Vorrichtung gemäß Anspruch 36, worin die Prozessorschaltung konfiguriert ist, ein Cluster assoziierter Zellsignalproteine zu identifizieren, wobei das Cluster eine Gruppe der Paare assoziierter Zellsignalproteine umfasst, wofür jedes Mitglied jedes Paares in mindestens einem anderen Paar der Gruppe vorhanden ist.

39. Vorrichtung gemäß Anspruch 38, worin die Prozessorschaltung konfiguriert ist, das Cluster assoziierter Zellsignalproteine zu identifizieren, indem
a) eine Clusterliste erstellt wird, die mit einem ersten Zellsignalprotein-Paar assoziiert ist, wobei die Clusterliste eine Identifikation des ersten Zellsignalprotein-Paares umfasst;
b) zu der Clusterliste eine Identifikation jedes Paares hinzugefügt wird, das zumindest ein Zellsignalprotein enthält, das auf dieser Clusterliste bereits vorhanden ist;
c) dieses Hinzufügen nach jeder Hinzufügung von Paaren zu der Clusterliste wiederholt wird, um zu der Clusterliste effektiv jedes Paar hinzuzufügen, das zumindest ein Zellsignalprotein enthält, das zumindest in einem Paar, das zu der Clusterliste hinzugefügt wurde, vorhanden ist;
d) aus der Clusterliste jedes Paar gestrichen wird, das zumindest ein Zellsignalprotein beinhaltet, das nicht in zumindest einem anderen Paar auf der Clusterliste zu finden ist; und
e) diese Streichung nach jeder Streichung von Paaren wiederholt wird, um effektiv jedes Paar, das zumindest ein Zellsignalprotein enthält, das nicht in zumindest einem anderen, nicht gestrichenen Paar auf der Clusterliste vorhanden ist, von der Clusterliste zu streichen.

40. Vorrichtung gemäß Anspruch 32, worin der Empfänger betätigbar ist, um Sets von Zellsignalprotein-Daten zu empfangen, wobei jedes Set die Datenwerte umfasst, wobei die Datenwerte Mengen jeweiliger korrespondierender Zellsignalproteine in biologischem Material entsprechend dem Set darstellen.

41. Vorrichtung gemäß Anspruch 40, worin die Prozessorschaltung konfiguriert ist, um als Vergleichswerte einen Coexpressions-Koeffizienten für jedes Paar der Zellsignalproteine zu erstellen, wobei jeder Coexpressions-Koeffizient einen Grad der Coexpression des einen Zellsignalproteins des Paares und des anderen Zellsignalproteins des Paares darstellt.

42. Vorrichtung gemäß Anspruch 41, worin die Prozessorschaltung konfiguriert ist, um jeden Coexpressions-Koeffizienten zu erstellen, indem
a) für jedes Set ein Differenzwert gleich einem absoluten Wert einer Differenz zwischen dem Datenwert entsprechend dem einen Zellsignalprotein und dem Datenwert entsprechend dem anderen Zellsignalprotein errechnet wird; und
b) diese Differenzwerte für jedes Set addiert werden, um eine Summe von Differenzwerten zu erhalten.

43. Vorrichtung gemäß Anspruch 42, worin die Prozessorschaltung konfiguriert ist, um die Summe durch die Anzahl der Sets zu dividieren, um den Coexpressions-Koeffizienten zu erhalten, der dem einen Zellsignalprotein und dem anderen Zellsignalprotein entspricht.

44. Vorrichtung gemäß Anspruch 42, worin die Prozessorschaltung konfiguriert ist, um die Zellsignalprotein-Paare zu identifizieren, indem jedes Zellsignalprotein-Paar identifiziert wird, das einen Coexpressions-Koeffizienten hat, der kleiner oder gleich einem Coexpressions-Schwellenwert ist.

45. Vorrichtung gemäß Anspruch 42, worin die Prozessorschaltung konfiguriert ist, um eine Liste coexprimierter Zellsignalprotein-Paare zu erstellen, wobei die Liste eine Identifikation jedes Zellsignalprotein-Paares umfasst, das einen Coexpressions-Koeffizienten kleiner oder gleich einem Coexpressions-Schwellenwert hat.

46. Vorrichtung gemäß Anspruch 45, worin die Prozessorschaltung konfiguriert ist, um eine Liste von Clustern coexprimierter Zellsignalprotein-Paare zu erstellen, wobei jedes Cluster eine Gruppe der Paare coexprimierter Zellsignalproteine umfasst, für welche jedes Mitglied jedes Paares in zumindest einem anderen Paar der Gruppe vorhanden ist.

47. Vorrichtung gemäß Anspruch 32, worin der Empfänger betätigbar ist, um Sets von Zellsignalprotein-Daten zu empfangen, wobei jedes Set Datenwerte umfasst, wobei die Werte die Phosphorylierungszustände der jeweiligen Zellsignalproteine in biologischem Material entsprechend dem Set angeben.

48. Vorrichtung gemäß Anspruch 47, worin die Prozessorschaltung konfiguriert ist, um als Vergleichswerte einen Coregulations-Koeffizienten für jedes Paar der Zellsignalproteine zu erstellen, wobei jeder Coregulations-Koeffizient einen Grad der Coregulation des einen Zellsignalproteins des Paares und des anderen Zellsignalproteins des Paares darstellt.

49. Vorrichtung gemäß Anspruch 48, worin die Prozessorschaltung konfiguriert ist, um jeden Coregulations-Koeffizienten zu erstellen, indem
a) für jedes Set ein Paar-Zustands-Wert als eine Funktion des Phosphorylierungszustands des einen Zellsignalproteins und des anderen Zellsignalproteins zugeordnet wird; und
b) die Paar-Zustands-Werte für jedes Set addiert werden, um eine Summe von Paar-Zustands-Werten zu erhalten.

50. Vorrichtung gemäß Anspruch 49, worin die Prozessorschaltung konfiguriert ist, um die Summe durch die Anzahl der Sets zu dividieren, um den Coregulations-Koeffizienten entsprechend dem einen Zellsignalprotein und dem anderen Zellsignalprotein zu erhalten.

51. Vorrichtung gemäß Anspruch 49, worin die Prozessorschaltung konfiguriert ist, um den Paar-Zustands-Wert zuzuordnen, indem
a) ein erster Paar-Zustands-Wert zugeordnet wird, wenn sich sowohl das eine Zellsignalprotein als auch das andere Zellsignalprotein in phosphoryliertem Zustand befinden;
b) ein zweiter Paar-Zustands-Wert zugeordnet wird, wenn sich sowohl das eine Zellsignalprotein als auch das andere Zellsignalprotein in dephosphoryliertem Zustand befinden, wobei der zweite Paar-Zustands-Wert geringer ist als der erste Paar-Zustands-Wert; und
c) ein dritter Paar-Zustands-Wert zugeordnet wird, wenn sich das eine Zellsignalprotein und das andere Zellsignalprotein in verschiedenen Phosphorlyierungszuständen befinden, wobei der dritte Paar-Zustands-Wert geringer ist als der zweite Paar-Zustands-Wert.

52. Vorrichtung gemäß Anspruch 49, worin die Prozessorschaltung konfiguriert ist, um die Zellsignalprotein-Paare zu identifizieren, indem jedes Zellsignalprotein-Paar identifiziert wird, das einen Coregulations-Koeffizienten hat, der größer ist als ein Coregulations-Schwellenwert.

53. Vorrichtung gemäß Anspruch 52, worin die Prozessorschaltung konfiguriert ist, um eine Liste coregulierter Zellsignalprotein-Paare zu erstellen, wobei die Liste eine Identifikation jedes Zellsignalprotein-Paares umfasst, das einen Coregulations-Koeffizienten hat, der größer als der Coregulations-Schwellenwert ist.

54. Vorrichtung gemäß Anspruch 53, worin die Prozessorschaltung konfiguriert ist, um eine Liste von Clustern coregulierter Zellsignalprotein-Paare zu erstellen, wobei jedes Cluster eine Gruppe der coregulierten Zellsignalprotein-Paare umfasst, für welche jedes Mitglied jedes Paares in zumindest einem anderen Paar der Gruppe vorhanden ist.

55. Vorrichtung gemäß Anspruch 32, worin die Prozessorschaltung konfiguriert ist, um als Vergleichswerte einen Verlinkungs-Koeffizienten für jedes Paar der Zellsignalproteine als eine Funktion eines Coexpressions-Koeffizienten, der einen Grad der Coexpression des einen Zellsignalproteins des Paares und des anderen Zellsignalproteins des Paares darstellt, und eines Coregulations-Koeffizienten, der einen Grad der Coregulation des einen Zellsignalproteins des Paares und des anderen Zellsignalproteins des Paares darstellt, zu erstellen, wobei jeder Verlinkungs-Koeffizient einen Grad der Assoziation zwischen dem einen Zellsignalprotein und dem anderen Zellsignalprotein darstellt.

56. Vorrichtung gemäß Anspruch 55, worin die Prozessorschaltung konfiguriert ist, um jeden Verlinkungs-Koeffizienten zu erstellen, indem für jedes Paar der Coregulations-Koeffizient durch den Coexpressions-Koeffizienten dividiert wird.

57. Vorrichtung gemäß Anspruch 56, worin die Prozessorschaltung konfiguriert ist, um eine Liste von verlinkten Zellsignalprotein-Paaren zu erstellen, wobei die Liste eine Identifikation jedes Zellsignalprotein-Paares mit einem Verlinkungs-Koeffizienten größer oder gleich einem Verlinkungs-Schwellenwert umfasst.

58. Vorrichtung gemäß Anspruch 56, worin die Prozessorschaltung konfiguriert ist, um zumindest einige der Zellsignalproteine mit jeweils gemeinsamen Signalwegen als Antwort auf die Verlinkungs-Koeffizienten zu assoziieren.

59. Vorrichtung gemäß Anspruch 58, worin die Prozessorschaltung konfiguriert ist, um die Zellsignalproteine mit den Wegen zu assoziieren, indem eine Gruppe der Zellsignalproteine identifiziert wird, für welche jeder Verlinkungs-Koeffizient, der jedes Zellsignalprotein mit jedem anderen Zellsignalprotein der Gruppe verlinkt, größer oder gleich einem Verlinkungs-Schwellenwert ist.

60. Vorrichtung gemäß Anspruch 59, worin die Prozessorschaltung konfiguriert ist, um die Gruppe zu identifizieren, indem
a) eine Verlinkungsliste erstellt wird, die ein erstes Zellsignalprotein umfasst;
b) zur Verlinkungsliste jedes andere Zellsignalprotein hinzugefügt wird, für welches der Verlinkungskoeffizient für das erste Zellsignalprotein und das andere Zellsignalprotein größer oder gleich dem Verlinkungs-Schwellenwert ist; und
c) von der Verlinkungsliste jedes Zellsignalprotein auf der Verlinkungsliste gestrichen wird, für welches der Verlinkungs-Koeffizient für jedes Zellsignalprotein und zumindest ein anderes Zellsignalprotein auf der Verlinkungsliste geringer ist als der Verlinkungs-Schwellenwert.

61. Vorrichtung gemäß Anspruch 59, worin die Prozessorschaltung konfiguriert ist, um Listen von gemeinsamen Signalwegen zu erstellen.

62. Vorrichtung gemäß Anspruch 32, weiters umfassend eine Messvorrichtung, die betätigbar ist, um die Datenwerte zu erstellen, die die physikalischen Eigenschaften der jeweiligen Zellsignalproteine darstellen.

63. Vorrichtung gemäß Anspruch 62, worin die Messvorrichtung einen Chemilumineszenz-Imager umfasst, der betätigbar ist, um Signale zu erzeugen, die für Proteine in einer einzigen Dimension in einem Elektrophorese-Gel repräsentativ sind.

64. Vorrichtung gemäß Anspruch 63, worin die Messvorrichtung weiters eine Elektrophoresevorrichtung umfasst, die betätigbar ist, um das Elektrophorese-Gel herzustellen.

65. Vorrichtung gemäß Anspruch 64, worin die Elektrophoresevorrichtung ein Messystem für die eindimensionale Natriumdodecylsulfat-Polyacrylamid-Gelektrophorese (SDS-PAGE) umfasst.

66. Computer-lesbares Medium für die Zur-Verfügung-Stellung von Instruktionen zur Ansteuerung einer programmierbaren Vorrichtung, um das Verfahren gemäß Anspruch 1 durchzuführen.

67. Computerdatensignal, verkörpert in einer Trägerwelle, wobei das Signal Codesegmente zur Ansteuerung einer programmierbaren Vorrichtung umfasst, um das Verfahren gemäß Anspruch 1 durchzuführen.

68. Computerprogramm, umfassend Codemittel, das, wenn es von einer Prozessorschaltung ausgeführt wird, alle Verfahrensschritte des Verfahrens gemäß Anspruch 1 durchführt.

## Revendications

1. Procédé d'analyse de protéines de signalisation cellulaire qui sont détectées dans un échantillon biologique unique pour identifier des protéines de signalisation cellulaire qui sont mutuellement associées dans des voies de signalisation communes, le procédé comprenant les étapes consistant à :
a. générer, dans des moyens matériels appropriés (15, 300, 302), des valeurs de données représentant les propriétés physiques des protéines de signalisation cellulaire détectées ;
b. recevoir dans un récepteur (12) lesdites valeurs de données représentant des propriétés physiques des protéines de signalisation cellulaire détectées ;
c. fournir un circuit de processeur (16) comprenant une mémoire (18), un processus d'analyse à exécuter par le circuit de processeur et des moyens de configuration (programme de coexpression 40 ; programme de corégulation 42 ; programme de liaison 44 ; sous-programme de groupement 46) adaptés pour diriger le circuit de processeur (16) afin d'exécuter le processus d'analyse ;
d. stocker dans ladite mémoire (18), pour chaque protéine de signalisation cellulaire détectée, les valeurs de données reçues des protéines de signalisation cellulaire dans des enregistrements de données adressables ;
e. exécuter le processus d'analyse dans le circuit de processeur (16) pour générer et stocker, dans la mémoire (18), une valeur de comparaison pour chaque paire possible de protéines de signalisation cellulaire détectées en réponse aux valeurs de données reçues des protéines de signalisation cellulaire, ladite valeur de comparaison étant un coefficient représentant un degré d'association entre une protéine de signalisation cellulaire de la paire et l'autre protéine de signalisation cellulaire de la paire ;
f. identifier des paires de protéines de signalisation cellulaire ayant des valeurs de comparaison répondant à une condition indicatrice d'une association entre les protéines de signalisation cellulaire ; et générer et stocker dans la mémoire une liste d'identifications de paires de protéines de signalisation cellulaire associées.

2. Procédé selon la revendication 1, dans lequel le processus d'analyse comprend un programme de coexpression.

3. Procédé selon la revendication 1, dans lequel le processus d'analyse comprend un programme de corégulation.

4. Procédé selon la revendication 1, comprenant en outre un moyen d'entrée pour une sélection par un utilisateur d'un processus d'analyse à partir d'une pluralité de processus d'analyse prédéterminés, le procédé comprenant la sélection dudit processus d'analyse parmi ladite pluralité de processus d'analyse, dans lequel lesdits moyens de configuration comprennent:
un moyen (40) adapté pour configurer le circuit de processeur pour générer, comme valeurs de comparaison, un coefficient de coexpression pour chaque paire des protéines de signalisation cellulaire, chaque coefficient de coexpression représentant un degré de coexpression d'une protéine de signalisation cellulaire de la paire et de l'autre protéine de signalisation cellulaire de la paire ;
un moyen (42) adapté pour configurer le circuit de processeur pour générer, comme valeurs de comparaison, un coefficient de corégulation pour chaque paire de protéines de signalisation cellulaire, chaque coefficient de corégulation représentant un degré de corégulation d'une protéine de signalisation cellulaire de la paire et de l'autre protéine de signalisation cellulaire de la paire ; et
un moyen (42) adapté pour configurer le circuit de processeur pour générer, comme valeur de comparaison, un coefficient de liaison, constitué du coefficient de corégulation divisé par le coefficient de coexpression, pour chaque paire de protéines de signalisation cellulaire, chaque coefficient de liaison représentant un degré d'association d'une protéine de signalisation cellulaire de la paire et de l'autre protéine de signalisation cellulaire de la paire.

5. Procédé selon l'une quelconque des revendications précédentes, dans lequel le stockage comprend le stockage desdites valeurs de comparaison dans une mémoire à accès aléatoire.

6. Procédé selon l'une quelconque des revendications précédentes, comprenant en outre la normalisation desdites valeurs de données par rapport à au moins une valeur de référence, avant la génération desdites valeurs de comparaison.

7. Procédé selon la revendication 1, dans lequel l'identification comprend la génération d'une liste de groupements de protéines de signalisation cellulaire associées.

8. Procédé selon l'une quelconque des revendications précédentes, dans lequel l'identification comprend en outre l'identification d'un groupement de protéines de signalisation cellulaire associées, ledit groupement comprenant un groupe desdites paires de protéines de signalisation cellulaire associées, pour lequel chaque élément de chacune desdites paires est présent dans au moins une autre paire dudit groupe.

9. Procédé selon la revendication 7, dans lequel l'identification de chacun desdits groupements consiste à :
a) générer une liste de groupements associée à une première paire de protéines de signalisation cellulaire, ladite liste de groupements comprenant une identification de ladite première paire de protéines de signalisation cellulaire ;
b) ajouter à ladite liste de groupements une identification de chacune desdites paires qui inclut au moins une protéine de signalisation cellulaire déjà présente dans ladite liste de groupements;
c) répéter ladite addition après chacune desdites additions de paires à ladite liste de groupements pour ajouter efficacement à ladite liste de groupements chacune desdites paires qui inclut au moins une protéine de signalisation cellulaire présente dans au moins une paire ajoutée à ladite liste de groupements ;
d) éliminer de ladite liste de groupements chacune desdites paires qui inclut au moins une protéine de signalisation cellulaire qui n'a pas été trouvée dans au moins une autre paire dans ladite liste de groupements ; et
e) répéter ladite élimination après chacune desdites éliminations de paires pour éliminer efficacement de ladite liste de groupements chacune desdites paires qui inclut au moins une protéine de signalisation cellulaire qui n'est pas présente dans au moins une autre paire non éliminée dans ladite liste de groupements.

10. Procédé selon la revendication 2 ou 4, dans lequel le processus d'analyse générant chacun desdits coefficients de coexpression consiste à :
a) calculer, pour chaque ensemble, une valeur de différence égale à une valeur absolue d'une différence entre ladite valeur de données correspondant à ladite une protéine de signalisation cellulaire et ladite valeur de données correspondant à ladite autre protéine de signalisation cellulaire ; et
b) additionner lesdites valeurs de différence pour chacun desdits ensembles pour produire une somme de valeurs de différence.

11. Procédé selon la revendication 10, dans lequel le processus d'analyse générant chacun desdits coefficients de coexpression comprend en outre la division de ladite somme par le nombre desdits ensembles pour générer ledit coefficient de coexpression pour ladite une protéine de signalisation cellulaire et ladite autre protéine de signalisation cellulaire.

12. Procédé selon la revendication 10, dans lequel l'identification de paires de protéines de signalisation cellulaire comprend l'identification de chaque paire de protéines de signalisation cellulaire ayant un coefficient de coexpression inférieur ou égal à une valeur de coexpression de seuil.

13. Procédé selon la revendication 10, dans lequel l'identification de paires de protéines de signalisation cellulaire comprend la génération d'une liste de paires de protéines de signalisation cellulaire coexprimées, ladite liste comprenant une identification de chacune desdites paires de protéines de signalisation cellulaire ayant un coefficient de coexpression inférieur ou égal à ladite valeur de coexpression de seuil.

14. Procédé selon la revendication 13, dans lequel l'identification de paires de protéines de signalisation cellulaire comprend en outre la génération d'une liste de groupements de paires de protéines de signalisation cellulaire coexprimées, chacun desdits groupements comprenant un groupe desdites paires de protéines de signalisation cellulaire coexprimées, pour lequel chaque élément de chaque paire est présent dans au moins une autre paire dudit groupe.

15. Procédé selon la revendication 1, dans lequel chaque ensemble de valeurs de données indique des états de phosphorylation de protéines de signalisation cellulaire respectives dans un matériau biologique correspondant audit ensemble.

16. Procédé selon la revendication 15, dans lequel la génération desdites valeurs de comparaison comprend la génération d'un coefficient de corégulation pour chaque paire desdites protéines de signalisation cellulaire, chacun desdits coefficients de corégulation représentant un degré de corégulation d'une protéine de signalisation cellulaire de ladite paire et de l'autre protéine de signalisation cellulaire de ladite paire.

17. Procédé selon la revendication 16, dans lequel la génération de chacun desdits coefficients de corégulation consiste à :
a) affecter, pour chaque ensemble, une valeur d'état de la paire en fonction des états de phosphorylation de ladite une protéine de signalisation cellulaire et de ladite autre protéine de signalisation cellulaire ; et
b) additionner lesdites valeurs d'état de la paire pour chacun desdits ensembles pour produire une somme de valeurs d'état de la paire.

18. Procédé selon la revendication 17, dans lequel la génération de chacun desdits coefficients de corégulation comprend en outre la division de ladite somme par le nombre desdits ensembles pour générer ledit coefficient de corégulation correspondant à ladite une protéine de signalisation cellulaire et à ladite autre protéine de signalisation cellulaire.

19. Procédé selon la revendication 17, dans lequel l'affectation d'une valeur d'état de la paire consiste à :
a) affecter une première valeur d'état de la paire lorsque ladite une protéine de signalisation cellulaire et ladite autre protéine de signalisation cellulaire sont toutes deux à l'état phosphorylé ;
b) affecter une deuxième valeur d'état de la paire lorsque ladite une protéine de signalisation cellulaire et ladite autre protéine de signalisation cellulaire sont toutes deux à l'état déphosphorylé, ladite deuxième valeur d'état de la paire étant inférieure à ladite première valeur d'état de la paire ; et
c) affecter une troisième valeur d'état de la paire lorsque ladite une protéine de signalisation cellulaire et ladite autre protéine de signalisation cellulaire sont dans des états de phosphorylation différents, ladite troisième valeur d'état de la paire étant inférieure à ladite deuxième valeur d'état de la paire.

20. Procédé selon la revendication 17, dans lequel l'identification de paires de protéines de signalisation cellulaire comprend l'identification de chaque paire de protéines de signalisation cellulaire ayant un coefficient de corégulation supérieur à une valeur de corégulation de seuil.

21. Procédé selon la revendication 20, dans lequel l'identification de paires de protéines de signalisation cellulaire comprend en outre la génération d'une liste de paires de protéines de signalisation cellulaire corégulées, ladite liste comprenant une identification de chacune desdites paires de protéines de signalisation cellulaire ayant un coefficient de corégulation supérieur à ladite valeur de corégulation de seuil.

22. Procédé selon la revendication 21, dans lequel l'identification de paires de protéines de signalisation cellulaire comprend en outre la génération d'une liste de groupements de paires de protéines de signalisation cellulaire corégulées, chacun desdits groupements comprenant un groupe desdites paires de protéines de signalisation cellulaire corégulées, pour lequel chaque élément de chaque paire est présent dans au moins une autre paire dudit groupe.

23. Procédé selon la revendication 1, dans lequel la génération d'une valeur de comparaison comprend la génération d'un coefficient de liaison pour chaque paire desdites protéines de signalisation cellulaire, en fonction d'un coefficient de coexpression représentant un degré de coexpression d'une protéine de signalisation cellulaire de ladite paire et de l'autre protéine de signalisation cellulaire de ladite paire, et d'un coefficient de corégulation représentant un degré de corégulation de ladite une protéine de signalisation cellulaire de ladite paire et de l'autre protéine de signalisation cellulaire de ladite paire, chacun desdits coefficients de liaison représentant un degré d'association entre ladite une protéine de signalisation cellulaire et ladite autre protéine de signalisation cellulaire.

24. Procédé selon la revendication 23, dans lequel la génération dudit coefficient de liaison comprend, pour chaque dite paire, la division dudit coefficient de corégulation par ledit coefficient de coexpression.

25. Procédé selon la revendication 24, comprenant en outre la génération d'une liste de paires de protéines de signalisation cellulaire liées, ladite liste comprenant une identification de chacune desdites paires de protéines de signalisation cellulaire ayant un coefficient de liaison supérieur ou égal à une valeur de liaison de seuil.

26. Procédé selon la revendication 24, comprenant en outre au moins certaines desdites protéines de signalisation cellulaire avec des voies de signalisation communes respectives, en réponse auxdits coefficients de liaison.

27. Procédé selon la revendication 26, dans lequel l'association comprend l'identification d'un groupe desdites protéines de signalisation cellulaire, pour lequel chacun desdits coefficients de liaison liant chaque protéine de signalisation cellulaire à chaque autre protéine de signalisation cellulaire dudit groupe est supérieur ou égal à une valeur de liaison de seuil.

28. Procédé selon la revendication 27, dans lequel l'identification dudit groupe consiste à :
a) générer une liste de liaisons comprenant une première protéine de signalisation cellulaire ;
b) ajouter à ladite liste de liaisons chacune desdites autres protéines de signalisation cellulaire, pour laquelle ledit coefficient de liaison pour ladite première protéine de signalisation cellulaire et ladite autre protéine de signalisation cellulaire est supérieur ou égal à ladite valeur de liaison de seuil ; et
c) éliminer de ladite liste de liaisons chacune desdites protéines de signalisation cellulaire de ladite liste de liaisons, pour laquelle ledit coefficient de liaison pour chacune desdites protéines de signalisation cellulaire et au moins une autre protéine de signalisation cellulaire sur ladite liste de liaisons est inférieur à ladite valeur de liaison de seuil.

29. Procédé selon la revendication 27, comprenant en outre la génération de listes desdites voies de signalisation communes.

30. Procédé selon la revendication 29, dans lequel lesdites valeurs de données sont représentatives de protéines dans une seule dimension dans un gel d'électrophorèse.

31. Procédé selon la revendication 30, comprenant en outre la production dudit gel d'électrophorèse.

32. Appareil adapté pour identifier des protéines de signalisation cellulaire associées selon le procédé de la revendication 1, lequel appareil comprend :
a) un récepteur qui est à même de recevoir des valeurs de données représentant des propriétés physiques de protéines de signalisation cellulaire respectives ; et
b) un circuit de processeur (16) en communication avec ledit récepteur, ledit circuit de processeur comprenant une mémoire (18) et ledit circuit de processeur (16) étant configuré pour exécuter un processus d'analyse pour :
i) générer et stocker dans la mémoire (18) une valeur de comparaison pour chaque paire desdites protéines de signalisation cellulaire en réponse auxdites valeurs de données ; et
ii) identifier des paires de protéines de signalisation cellulaire ayant des valeurs de comparaison répondant à une condition indicatrice d'une association entre les protéines de signalisation cellulaire ; et générer et stocker dans la mémoire (18) une liste d'identifications de paires de protéines de signalisation cellulaire associées.

33. Appareil selon la revendication 32, comprenant en outre un moyen d'entrée pour une sélection par un utilisateur dudit processus d'analyse parmi une pluralité de processus d'analyse prédéterminés, dans lequel ledit moyen de configuration comprend :
un moyen (40) adapté pour configurer le circuit de processeur pour générer, comme valeurs de comparaison, un coefficient de coexpression pour chaque paire de protéines de signalisation cellulaire, chaque coefficient de coexpression représentant un degré de coexpression d'une protéine de signalisation cellulaire de la paire et de l'autre protéine de signalisation cellulaire de la paire ;
un moyen (42) adapté pour configurer le circuit de processeur pour générer, comme valeurs de comparaison, un coefficient de corégulation pour chaque paire de protéines de signalisation cellulaire, chaque coefficient de corégulation représentant un degré de corégulation d'une protéine de signalisation cellulaire de la paire et de l'autre protéine de signalisation cellulaire de la paire ; et
un moyen (42) adapté pour configurer le circuit de processeur pour générer, comme valeur de comparaison, un coefficient de liaison, constitué du coefficient de corégulation divisé par le coefficient de coexpression, pour chaque paire de protéines de signalisation cellulaire, chaque coefficient de liaison représentant un degré d'association d'une protéine de signalisation cellulaire de la paire et de l'autre protéine de signalisation cellulaire de la paire.

34. Appareil selon la revendication 33, dans lequel ladite mémoire comprend une mémoire à accès aléatoire.

35. Appareil selon la revendication 34, dans lequel ledit circuit de processeur est configuré pour normaliser lesdites valeurs de données par rapport à au moins une valeur de référence, avant la génération desdites valeurs de comparaison.

36. Appareil selon la revendication 34, dans lequel ledit circuit de processeur est configuré pour générer une liste de paires de protéines de signalisation cellulaire associées.

37. Appareil selon la revendication 34, dans lequel ledit circuit de processeur est configuré pour générer une liste de groupements de protéines de signalisation cellulaire associées.

38. Appareil selon la revendication 36, dans lequel ledit circuit de processeur est configuré pour identifier un groupement de protéines de signalisation cellulaire associées, ledit groupement comprenant un groupe desdites paires de protéines de signalisation cellulaire associées, pour lequel chaque élément de chacune desdites paires est présent dans au moins une autre paire dudit groupe.

39. Appareil selon la revendication 38, dans lequel ledit circuit de processeur est configuré pour identifier ledit groupement de protéines de signalisation cellulaire associées,
a) en générant une liste de groupements associée à une première paire de protéines de signalisation cellulaire, ladite liste de groupements comprenant une identification de ladite première paire de protéines de signalisation cellulaire ;
b) en ajoutant à ladite liste de groupements une identification de chacune desdites paires qui inclut au moins une protéine de signalisation cellulaire déjà présente dans ladite liste de groupements ;
c) en répétant ladite addition après chacune desdites additions de paires à ladite liste de groupements pour ajouter efficacement à ladite liste de groupements chacune desdites paires qui inclut au moins une protéine de signalisation cellulaire présente dans au moins une paire ajoutée à ladite liste de groupements ;
d) en éliminant de ladite liste de groupements chacune desdites paires qui inclut au moins une protéine de signalisation cellulaire qui n'a pas été trouvée dans au moins une autre paire non éliminée dans ladite liste de groupements ; et
e) en répétant ladite élimination après chacune desdites éliminations de paires pour éliminer efficacement de ladite liste de groupements chacune desdites paires qui inclut au moins une protéine de signalisation cellulaire qui n'est pas présente dans au moins une autre paire non éliminée dans ladite liste de groupements.

40. Appareil selon la revendication 32, dans lequel ledit récepteur est à même de recevoir des ensembles de données de protéines de signalisation cellulaire, chaque ensemble comprenant lesdites valeurs de données, lesdites valeurs de données représentant des quantités de protéines de signalisation cellulaire correspondantes respectives dans un matériau biologique correspondant audit ensemble.

41. Appareil selon la revendication 40, dans lequel ledit circuit de processeur est configuré pour générer, comme valeurs de comparaison, un coefficient de coexpression pour chaque paire desdites protéines de signalisation cellulaire, chacun desdits coefficients de coexpression représentant un degré de coexpression d'une protéine de signalisation cellulaire de ladite paire et de l'autre protéine de signalisation cellulaire de ladite paire.

42. Appareil selon la revendication 41, dans lequel ledit circuit de processeur est configuré pour générer chacun desdits coefficients de coexpression,
a) en calculant, pour chaque ensemble, une valeur de différence égale à une valeur absolue d'une différence entre ladite valeur de données correspondant à ladite une protéine de signalisation cellulaire et ladite valeur de données correspondant à ladite autre protéine de signalisation cellulaire; et
b) en additionnant lesdites valeurs de différence pour chacun desdits ensembles afin de produire une somme de valeurs de différence.

43. Appareil selon la revendication 42, dans lequel ledit circuit de processeur est configuré pour diviser ladite somme par le nombre desdits ensembles afin de générer ledit coefficient de coexpression correspondant à ladite une protéine de signalisation cellulaire et à ladite autre protéine de signalisation cellulaire.

44. Appareil selon la revendication 42, dans lequel ledit circuit de processeur est configuré pour identifier lesdites paires de protéines de signalisation cellulaire, en identifiant chaque paire de protéines de signalisation cellulaire ayant un coefficient de coexpression inférieur ou égal à une valeur de coexpression de seuil.

45. Appareil selon la revendication 42, dans lequel ledit circuit de processeur est configuré pour générer une liste de paires de protéines de signalisation cellulaire coexprimées, ladite liste comprenant une identification de chacune desdites paires de protéines de signalisation cellulaire ayant un coefficient de coexpression inférieur ou égal à ladite valeur de coexpression de seuil.

46. Appareil selon la revendication 45, dans lequel ledit circuit de processeur est configuré pour générer une liste de groupements de paires de protéines de signalisation cellulaire coexprimées, chacun desdits groupements comprenant un groupe desdites paires de protéines de signalisation cellulaire coexprimées, pour lequel chaque élément de chaque paire est présent dans au moins une autre paire dudit groupe.

47. Appareil selon la revendication 32, dans lequel ledit récepteur est à même de recevoir des ensembles de données de protéines de signalisation cellulaire, chaque ensemble comprenant lesdites valeurs de données, lesdites valeurs de données indiquant des états de phosphorylation de protéines de signalisation cellulaire respectives dans un matériau biologique correspondant audit ensemble.

48. Appareil selon la revendication 47, dans lequel ledit circuit de processeur est configuré pour générer, comme valeurs de comparaison, un coefficient de corégulation pour chaque paire desdites protéines de signalisation cellulaire, chacun desdits coefficients de corégulation représentant un degré de corégulation d'une protéine de signalisation cellulaire de ladite paire et de l'autre protéine de signalisation cellulaire de ladite paire.

49. Appareil selon la revendication 48, dans lequel ledit circuit de processeur est configuré pour générer chacun desdits coefficients de corégulation,
a) en affectant, pour chaque ensemble, une valeur d'état de la paire en fonction des états de phosphorylation de ladite une protéine de signalisation cellulaire et de ladite autre protéine de signalisation cellulaire ; et
b) en additionnant lesdites valeurs d'état de la paire pour chacun desdits ensembles afin de produire une somme de valeurs d'état de la paire.

50. Appareil selon la revendication 49, dans lequel ledit circuit de processeur est configuré pour diviser ladite somme par le nombre desdits ensembles afin de générer ledit coefficient de corégulation correspondant à ladite une protéine de signalisation cellulaire et à ladite autre protéine de signalisation cellulaire.

51. Appareil selon la revendication 49, dans lequel ledit circuit de processeur est configuré pour affecter ladite valeur d'état de la paire,
a) en affectant une première valeur d'état de la paire lorsque ladite une protéine de signalisation cellulaire et ladite autre protéine de signalisation cellulaire sont toutes deux à l'état phosphorylé ;
b) en affectant une deuxième valeur d'état de la paire lorsque ladite une protéine de signalisation cellulaire et ladite autre protéine de signalisation cellulaire sont toutes deux à l'état déphosphorylé, ladite deuxième valeur d'état de la paire étant inférieure à ladite première valeur d'état de la paire ; et
c) en affectant une troisième valeur d'état de la paire lorsque ladite une protéine de signalisation cellulaire et ladite autre protéine de signalisation cellulaire sont dans des états de phosphorylation différents, ladite troisième valeur d'état de la paire étant inférieure à ladite deuxième valeur d'état de la paire.

52. Appareil selon la revendication 49, dans lequel ledit circuit de processeur est configuré pour identifier lesdites paires de protéines de signalisation cellulaire en identifiant chaque paire de protéines de signalisation cellulaire ayant un coefficient de corégulation supérieur à une valeur de corégulation de seuil.

53. Appareil selon la revendication 52, dans lequel ledit circuit de processeur est configuré pour générer une liste de paires de protéines de signalisation cellulaire corégulées, ladite liste comprenant une identification de chacune desdites paires de signalisation cellulaire ayant un coefficient de corégulation supérieur à ladite valeur de corégulation de seuil.

54. Appareil selon la revendication 53, dans lequel ledit circuit de processeur est configuré pour générer une liste de groupements de paires de protéines de signalisation cellulaire corégulées, chacun desdits groupements comprenant un groupe desdites paires de protéines de signalisation cellulaire corégulées, pour lequel chaque élément de chaque paire est présent dans au moins une autre paire dudit groupe.

55. Appareil selon la revendication 32, dans lequel ledit circuit de processeur est configuré pour générer, comme valeurs de comparaison, un coefficient de liaison pour chaque paire desdites protéines de signalisation cellulaire, en fonction d'un coefficient de coexpression représentant un degré de coexpression d'une protéine de signalisation cellulaire de ladite paire et de l'autre protéine de signalisation cellulaire de ladite paire, et d'un coefficient de corégulation représentant un degré de corégulation de ladite une protéine de signalisation cellulaire de ladite paire et de ladite autre protéine de signalisation cellulaire de ladite paire, chacun desdits coefficients de liaison représentant un degré d'association entre ladite une protéine de signalisation cellulaire et ladite autre protéine de signalisation cellulaire.

56. Appareil selon la revendication 55, dans lequel ledit circuit de processeur est configuré pour générer chacun desdits coefficients de liaison en divisant, pour chacune desdites paires, ledit coefficient de corégulation par ledit coefficient de coexpression.

57. Appareil selon la revendication 56, dans lequel ledit circuit de processeur est configuré pour générer une liste de paires de protéines de signalisation cellulaire liées, ladite liste comprenant une identification de chacune desdites paires de protéines de signalisation cellulaire ayant un coefficient de liaison supérieur ou égal à une valeur de liaison de seuil.

58. Appareil selon la revendication 56, dans lequel ledit circuit de processeur est configuré pour associer au moins certaines desdites protéines de signalisation cellulaire avec des voies de signalisation communes respectives, en réponse auxdits coefficients de liaison.

59. Appareil selon la revendication 58, dans lequel ledit circuit de processeur est configuré pour associer lesdites protéines de signalisation cellulaire avec lesdites voies en identifiant un groupe desdites protéines de signalisation cellulaire, pour lequel chacun desdits coefficients de liaison liant chacune desdites protéines de signalisation cellulaire à chaque autre protéine de signalisation cellulaire dudit groupe est supérieur ou égal à une valeur de liaison de seuil.

60. Appareil selon la revendication 59, dans lequel ledit circuit de processeur est configuré pour identifier ledit groupe,
a) en générant une liste de liaisons comprenant une première protéine de signalisation cellulaire ;
b) en ajoutant à ladite liste de liaisons chacune desdites protéines de signalisation cellulaire, pour laquelle ledit coefficient de liaison pour ladite première protéine de signalisation cellulaire et ladite autre protéine de signalisation cellulaire est supérieur ou égal à ladite valeur de liaison de seuil; et
c) en éliminant de ladite liste de liaisons chacune desdites protéines de signalisation cellulaire sur ladite liste de liaisons, pour laquelle ledit coefficient de liaison pour chacune desdites protéines de signalisation cellulaire et au moins une autre protéine de signalisation cellulaire de ladite liste de liaisons est inférieur à ladite valeur de liaison de seuil.

61. Appareil selon la revendication 59, dans lequel ledit circuit de processeur est configuré pour générer des listes desdites voies de signalisation communes.

62. Appareil selon la revendication 32, comprenant en outre un dispositif de mesure qui est à même de générer lesdites valeurs de données représentant lesdites propriétés physiques desdites protéines de signalisation cellulaire respectives.

63. Appareil selon la revendication 62, dans lequel ledit dispositif de mesure comprend un dispositif d'imagerie à chimioluminescence, qui peut être à même de générer des signaux représentatifs de protéines dans une seule dimension dans un gel d'électrophorèse.

64. Appareil selon la revendication 63, dans lequel ledit dispositif de mesure comprend en outre un appareil d'électrophorèse qui est à même de générer ledit gel d'électrophorèse.

65. Appareil selon la revendication 64, dans lequel ledit appareil d'électrophorèse comprend un système de mesure par électrophorèse sur gel unidimensionnel de dodécylsulfate de sodium et de polyacrylamide (SDS-PAGE).

66. Support lisible par ordinateur pour fournir des instructions destinées à diriger un dispositif programmable pour exécuter le procédé de la revendication 1.

67. Signal de données informatique mis en oeuvre dans une onde porteuse, le signal comprenant des segments de code pour diriger un dispositif programmable afin d'exécuter le procédé de la revendication 1.

68. Programme informatique comprenant un moyen de codage qui, lorsqu'il est exécuté par un circuit de processeur, effectue toutes les étapes du procédé de la revendication 1.
